(19) Europäisches Patentamt
European Patent Office
Office européen des brevets

(11) EP 3 590 404 A1

(12) EUROPEAN PATENT APPLICATION
published in accordance with Art. 153(4) EPC

(43) Date of publication:
08.01.2020 Bulletin 2020/02

(51) Int Cl.:
A61B 1/00 (2006.01)     A61B 1/045 (2006.01)

(21) Application number: 18760655.3

(22) Date of filing: 14.02.2018

(86) International application number:
PCT/JP2018/004982

(87) International publication number:
WO 2018/159288 (07.09.2018 Gazette 2018/36)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD TN

(30) Priority: 28.02.2017 JP 2017037025

(71) Applicant: Sony Corporation
Tokyo 108-0075 (JP)

(72) Inventors:
• ZHU, Yiwen
  Tokyo 108-0075 (JP)
• KAMIO, Kazunori
  Tokyo 108-0075 (JP)
• KAWATA, Satoshi
  Tokyo 108-0075 (JP)

(74) Representative: D Young & Co LLP
120 Holborn
London EC1N 2DY (GB)

(54) IMAGE PROCESSING APPARATUS, IMAGE FORMING METHOD, AND PROGRAM

(57) An apparatus and a method are provided for generating a superimposed image of a visible light image and a fluorescence image having a noise level which substantially coincides with a noise level of the visible light image. A fluorescence image noise reduction unit which executes noise reduction processing on a fluorescence image and generates a noise reduced fluorescence image, an image superimposing unit which generates a superimposed image of a visible light image and a noise reduced fluorescence image according to a set superimposing rate, and a fluorescence image noise reduction intensity calculation unit which calculates a noise reduction intensity which is applied to the noise reduction processing on the fluorescence image are included. Even in a case where the superimposing rate is changed, the fluorescence image noise reduction intensity calculation unit calculates a noise reduction intensity having a value which makes a noise level of the noise reduced fluorescence image substantially coincide with a noise level of the visible light image forming the superimposed image.

FIG. 2

EP 3 590 404 A1

**Description**

TECHNICAL FIELD

The present disclosure relates to an image processing apparatus, an image processing method, and a program. In particular, the present disclosure relates to an image processing apparatus, an image processing method, and a program which execute image processing using a visible light image and a fluorescence image.

BACKGROUND ART

Recently, an endoscope for capturing an image in a living body has increasingly used a fluorescence image different from a visible light image in addition to the visible light image which is a normal color image.

The fluorescence image is an image obtained by, for example, irradiating excitation light having a specific wavelength band and imaging fluorescence included in light reflected by a substance in a living body.

The fluorescence image can express, for example, a difference in the intensity according to a lesion in the living body and the like, and it is possible to effectively analyze a progress in disease and the like by using the fluorescence image.

However, there is a problem in that the fluorescence image has a lower image quality than the visible light image, and a combination of the visible light image and the fluorescence image is often used.

Note that an endoscope device using the visible light image and the fluorescence image is described, for example, in Patent Document 1 (Japanese Patent Application Laid-Open No. 2013-212323) and the like.

Patent Document 1 (Japanese Patent Application Laid-Open No. 2013-212323) discloses a configuration in which a fluorescence image is superimposed with a white image (visible light image) when a fluorescence image obtained by an imaging element for capturing the fluorescence image has a high image quality and a marker indicating a position of a lesion portion is superimposed on a white image (visible light image) when the fluorescence image has a low image quality. With this configuration, an image observer can easily confirm the position of the lesion portion.

This Patent Document 1 discloses a configuration for generating a superimposed image of the fluorescence image and the white image (visible light image).

However, for example, regarding medical devices, in order to reliably confirm a lesion portion, a doctor for observing an image and the like has required to observe an image of visible light alone, an image of fluorescence image alone, and in addition, various images in which superimposing rates of the visible light image and the fluorescence image are changed.

However, in general, the visible light image is a clear image with a lower noise level. Whereas, the fluorescence image is an unclear image with a higher noise level.

In a case where two images having different noise levels like these are superimposed and displayed, the following problems occur.

For example, when a superimposed image obtained by superimposing the fluorescence image on the visible light image is displayed after the processing for displaying the visible light image, a case occurs where a point and a line caused by noise included in the fluorescence image which do not exist in the visible light image are suddenly displayed on a display device.

For example, in a case where an operation unit for changing a superimposing rate of the visible light image and the fluorescence image is provided in the display device, an observer such as a doctor operates the operation unit, and the superimposing rate of the visible light image and the fluorescence image is sequentially changed so as to continuously observe images having various superimposing rates, there is a case where the point and the line caused by the noise of the fluorescence image are displayed or disappeared according to the change in the superimposing rate.

There is a problem in that, when such a situation occurs, it is difficult for an image observer such as a doctor to determine whether the point and the line are subject images of the lesion portion and the like or the noise, and the image observer cannot make accurate diagnosis.

CITATION LIST

PATENT DOCUMENT

Patent Document 1: Japanese Patent Application Laid-Open No. 2013-212323

SUMMARY OF THE INVENTION

PROBLEMS TO BE SOLVED BY THE INVENTION

The present disclosure has been made, for example, in consideration of the programs described above, and an object of the present disclosure is to provide an image processing apparatus, an image processing method, and a program which have a configuration for displaying an image obtained by superimposing a visible light image and a fluorescence image and can control noise levels of the two images and display the obtained image.

An object of the embodiment of the present disclosure is to provide an image processing apparatus, an image processing method, and a program which can generate and display a superimposed image in which the noise levels of the visible light image and the fluorescence image included in the superimposed image substantially coincide with each other even when the superimposing rate of the visible light image and the fluorescence image changes.

SOLUTIONS TO PROBLEMS

A first aspect of the present disclosure is an image processing apparatus which includes
a fluorescence image noise reduction unit which executes noise reduction processing on a fluorescence image and generates a noise reduced fluorescence image, and
an image superimposing unit which generates a superimposed image of a visible light image and the noise reduced fluorescence image,
in which
the noise reduction processing is processing for adjusting a noise level of the fluorescence image to be closer to a noise level of the visible image forming the superimposed image.

Moreover, a second aspect of the present disclosure is
an image processing method executed by an image processing apparatus including
a fluorescence image noise reduction step for executing noise reduction processing on a fluorescence image and generating a noise reduced fluorescence image by a fluorescence image noise reduction unit and
an image superimposing step for generating a superimposed image of a visible light image and the noise reduced fluorescence image by an image superimposing unit, in which
the noise reduction processing executed in the fluorescence image noise reduction step is processing for adjusting a noise level of the fluorescence image to be closer to a noise level of the visible image forming the superimposed image.

Moreover, a third aspect of the present disclosure is
a program for causing an image processing apparatus to execute image processing including
a fluorescence image noise reduction step for causing a fluorescence image noise reduction unit to execute noise reduction processing on a fluorescence image and generate a noise reduced fluorescence image and
an image superimposing step for causing an image superimposing unit to generate a superimposed image of a visible light image and the noise reduced fluorescence image, in which
the noise reduction processing executed in the fluorescence image noise reduction step is processing for adjusting a noise level of the fluorescence image to be closer to a noise level of the visible image forming the superimposed image.

Note that, for example, a program according to the present disclosure can be provided by a storage medium and a communication medium which provide the program in a computer-readable format to an information processing apparatus and a computer system which can execute various program codes. The information processing apparatus and the computer system can realize processing according to the program by providing these programs in a computer-readable form.

Other purpose, characteristics, and advantages of the present disclosure would be obvious by the detailed description based on the embodiments of the present disclosure as described later and the attached drawings. Note that, in the present specification, the system is a logical group configuration of a plurality of devices, and the devices of the configuration are not limited to be housed in the same casing.

EFFECTS OF THE INVENTION

According to one embodiment of the present disclosure, an apparatus and a method are realized for generating a superimposed image of a visible light image and a fluorescence image having a noise level which substantially coincides with a noise level of the visible light image.

Specifically, for example, a fluorescence image noise reduction unit which executes noise reduction processing on the fluorescence image and generates a noise reduced fluorescence image, an image superimposing unit which generates a superimposed image of the visible light image and the noise reduced fluorescence image according to the set superimposing rate, and a fluorescence image noise reduction intensity calculation unit which calculates a noise reduction intensity to be applied to the noise reduction processing on the fluorescence image by the fluorescence image noise reduction unit. Even in a case where the superimposing rate is changed, the fluorescence image noise reduction intensity calculation unit calculates a noise reduction intensity having a value which makes a noise level of the noise reduced fluorescence image substantially coincide with a noise level of the visible light image forming the superimposed image.

With these configuration and processing, an apparatus and a method are realized for generating a superimposed image of a visible light image and a fluorescence image having a noise level which substantially coincides with a noise level of the visible light image.

Note that the effects described herein are only exemplary and not limited to these. Furthermore, there may be an additional effect.

BRIEF DESCRIPTION OF DRAWINGS

Fig. 1 is a diagram for explaining a fluorescence image.
Fig. 2 is a diagram for explaining an exemplary configuration of an image processing apparatus.
Fig. 3 is a diagram for explaining a configuration and processing of a superimposing rate setting unit.
Fig. 4 is a diagram for explaining processing executed by a noise level estimation unit.
Fig. 5 is a diagram for explaining processing executed by a fluorescence image noise reduction intensity calculation unit.
Fig. 6 is a diagram for explaining processing executed by a fluorescence image noise reduction unit.
Fig. 7 is a diagram for explaining processing executed by an image superimposing unit.
Fig. 8 is a diagram illustrating a flowchart for explaining a processing sequence executed by the image processing apparatus.
Fig. 9 is a diagram for explaining an exemplary configuration of the image processing apparatus.
Fig. 10 is a diagram for explaining processing executed by the fluorescence image noise reduction unit.
Fig. 11 is a diagram for explaining a LUT of the fluorescence image noise reduction unit.
Fig. 12 is a diagram illustrating a flowchart for explaining a processing sequence executed by the image processing apparatus.
Fig. 13 is a diagram for explaining an exemplary configuration of the image processing apparatus.
Fig. 14 is a diagram for explaining processing executed by the fluorescence image noise reduction unit.
Fig. 15 is a diagram for explaining the LUT of the fluorescence image noise reduction unit.
Fig. 16 is a diagram for explaining processing executed by a class classification processing unit of the fluorescence image noise reduction unit.
Fig. 17 is a diagram illustrating a flowchart for explaining a processing sequence executed by the image processing apparatus.
Fig. 18 is a diagram for explaining an exemplary configuration of the image processing apparatus.
Fig. 19 is a diagram for explaining processing executed by the fluorescence image noise reduction intensity calculation unit.
Fig. 20 is a diagram illustrating a flowchart for explaining a processing sequence executed by the image processing apparatus.
Fig. 21 is a diagram illustrating an exemplary hardware configuration of the image processing apparatus.

MODE FOR CARRYING OUT THE INVENTION

Hereinafter, an image processing apparatus, an image processing method, and a program according to the present disclosure will be described in detail with reference to the drawings. Note that the description will be made according to the following items.

1. Outline of fluorescence image
2. Configuration and processing of first embodiment of image processing apparatus according to present disclosure
3. Sequence of processing executed by image processing apparatus
4. Configuration and processing of second embodiment of image processing apparatus according to present disclosure

4-1. Exemplary configuration for determining filter size on the basis of noise reduction intensity
4-2. Exemplary configuration for determining filter size on the basis of learning data based on noise reduction intensity and image feature amount

5. Configuration and processing of third embodiment of image processing apparatus according to present disclosure
6. Exemplary hardware configuration of image processing apparatus
7. Summary of configuration of present disclosure

[1. Outline of fluorescence image]

First, an outline of a fluorescence image will be described.

As described above, an endoscope for capturing an image in a living body has increasingly used a fluorescence image in addition to a visible light image which is a normal color image.

The fluorescence image is an image obtained by irradiating excitation light having a specific wavelength and imaging fluorescence included in light reflected by a substance in the living body.

The fluorescence image can express, for example, a difference in the intensity according to a lesion in the living body and the like, and it is possible to effectively analyze a progress in disease and the like by using the fluorescence image.

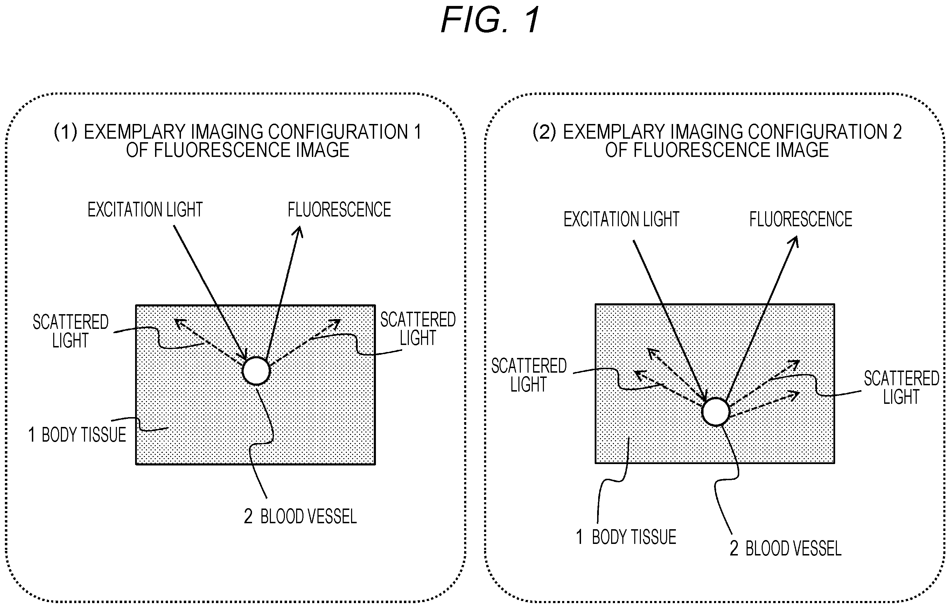

With reference to Fig. 1, an imaging configuration of the fluorescence image will be described.

The fluorescence image is an image obtained by irradiating excitation light having a specific wavelength and inputting fluorescence output from a body tissue such as a blood vessel, for example, to an imaging element.

Fig. 1(1) illustrates an exemplary configuration for imaging a blood vessel 2 in a relatively shallow portion in a body tissue 1, and Fig. 1(2) illustrates an exemplary configuration for imaging the blood vessel 2 in a relatively deep portion in the body tissue 1.

When the blood vessel is irradiated with the excitation light, a plurality of scattered light beams is generated. In particular, there is a problem in that, in the deep portion of the body tissue 1, more scattered light is generated, and as a result, noise of the fluorescence image imaged by the imaging element increases.

As described above, for example, regarding medical devices, in order to reliably confirm a lesion portion, a doctor for observing an image and the like has required to observe an image of visible light alone, an image of fluorescence image alone, and in addition, various images in which superimposing rates of the visible light image and the fluorescence image are changed.

Specifically, for example, a configuration is required in which an operation unit for changing the superimposing rates of the visible light image and the fluorescence image is provided in a display device, an observer such as a doctor operates the operation unit, and the superimposing rates of the visible light image and the fluorescence image are sequentially changed so as to continuously observe various images.

However, as described above, a number of visible light images are clear images with a lower noise level. Whereas, the fluorescence image is an unclear image with a higher noise level.

When the superimposing rates of the visible light image and the fluorescence image having different noise levels like these are sequentially changed and images with various superimposing rates are continuously displayed, there is a case where a point and a line caused by the noise in the fluorescence image are displayed or disappeared according to the change in the superimposing rate.

When such a situation occurs, it is difficult for an image observer such as a doctor to determine whether the point and the line are subject images of the lesion portion and the like or the noise, and the image observer cannot make accurate diagnosis.

Hereinafter, a configuration that solves the above problems, in other words, a configuration and processing of the present disclosure which can control the noise level of the image and display the image in a case where a superimposed image of the visible light image and the fluorescence image is displayed.

[2. Configuration and processing of first embodiment of image processing apparatus according to present disclosure]

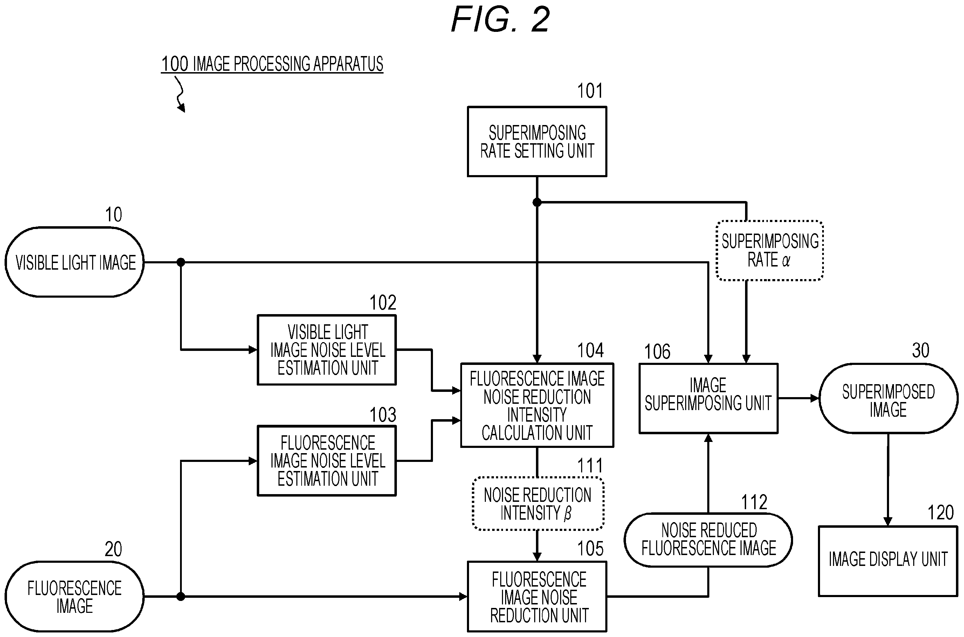

A configuration and processing of a first embodiment of the image processing apparatus according to the present disclosure will be described with reference to Fig. 2 and subsequent drawings.

Fig. 2 is a block diagram illustrating an exemplary configuration of an image processing apparatus 100 according to the first embodiment of the present disclosure.

Note that, the image processing apparatus according to the present disclosure can be realized by, specifically, for example, an imaging device, an information processing apparatus such as a PC which receives an image captured by the imaging device and executes image processing, and the like.

The image processing apparatus 100 illustrated in Fig. 2 receives a visible light image 10 and a fluorescence image 20 and generates a superimposed image 30 obtained by superimposing the two images and outputs the superimposed image 30. The superimposed image 30 is, for example, output to and displayed by an image display device 120 having an image display unit (display).

The image processing apparatus 100 illustrated in Fig. 2 has a configuration for controlling a noise level of the superimposed image 30 obtained by superimposing the visible light image 10 and the fluorescence image 20. Specifically, for example, a configuration is included which can lower the noise level of the fluorescence image 20 to the noise level of the visible light image 10 which is a superimposing target and superimposes the fluorescence image 20 and the visible light image 10.

Note that, the description will be made below as assuming that the image processing apparatus 100 has a configuration for executing superimposition processing on the visible light image 10 and the fluorescence image 20. However, a combination of images to be superimposed by the image processing apparatus according to the present disclosure is not limited to a combination of the visible light image and the fluorescence image, and various images having different noise levels can be applied.

For example, the present disclosure can be applied to superimposition processing of a visible light image and a (far) infrared image, superimposition processing of a visible light image with a lower noise level and a visible light image with a higher noise level, and the like, and in addition, the configuration and the processing according to the present disclosure can be applied to various image superimposition processing.

In the following description, as an example of the image processing apparatus 100 according to the present disclosure, an embodiment for executing the superimposition processing of the visible light image 10 and the fluorescence image 20 will be described.

The visible light image 10 and the fluorescence image 20 input to the image processing apparatus 100 illustrated in Fig. 2 are images of the same subject.

For example, the image processing apparatus 100 may have a configuration having an imaging unit which executes processing for imaging the visible light image and the fluorescence image, and the image processing apparatus 100 may have a configuration which does not have the imaging unit and receives the visible light image and the fluorescence image captured by the imaging device.

Note that, in a case where the two images have a positional gap or the like, positioning processing to which existing technology is applied is executed in advance.

In other words, the visible light image 10 and the fluorescence image 20 illustrated in Fig. 2 are two images which are obtained by imaging the same subject and on which positioning has been performed.

As illustrated in Fig. 2, the image processing apparatus 100 includes a superimposing rate setting unit 101, a visible light image noise level estimation unit 102, a fluorescence image noise level estimation unit 103, a fluorescence image noise reduction intensity calculation unit 104, a fluorescence image noise reduction unit 105, and an image superimposing unit 106.

First, an outline of processing executed by each component of the image processing apparatus 100 will be described, and specific processing of each component will be described later in detail.

The superimposing rate setting unit 101 has an operation unit which can be operated by an image observer such as a doctor, for example, and adjusts a superimposing rate which is a ratio between a visible light image component and a fluorescence image component forming the superimposed image 30.

The visible light image noise level estimation unit 102 and the fluorescence image noise level estimation unit 103 respectively estimate noise levels of noise included in the visible light image 10 and the fluorescence image 20.

The fluorescence image noise reduction intensity calculation unit 104 calculates a noise level reduction intensity $\beta$, 111 which is applied to noise reduction processing executed by the fluorescence image noise reduction unit 105 on the fluorescence image 20.

For example, even when the superimposing rates of the visible light image and the fluorescence image change, the fluorescence image noise reduction intensity calculation unit 104 calculates the noise level reduction intensity $\beta$, 111 according to the superimposing rate so that the noise levels of the visible light image and the fluorescence image included in the superimposed image are substantially coincide with each other.

The fluorescence image noise reduction unit 105 executes noise level reduction processing on the fluorescence image 20 according to the noise level reduction intensity $\beta$, 111 calculated by the fluorescence image noise reduction intensity calculation unit 104.

The image superimposing unit 106 superimposes the visible light image 10 with a noise reduced fluorescence image 112 generated by the fluorescence image noise reduction unit 105 and generates the superimposed image 30.

The generated superimposed image 30 is displayed on the image display unit 120.

First, the outline of the processing executed by each component of the image processing apparatus 100 has been described.

Next, the specific processing of each component will be described in detail.

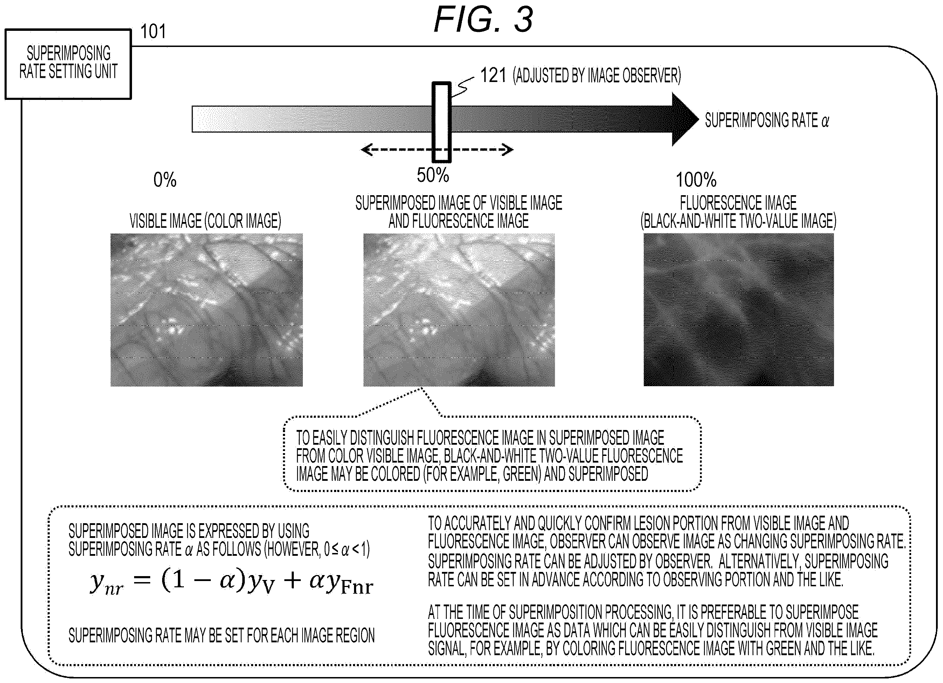

First, the superimposing rate setting unit 101 will be described with reference to Fig. 3.

As described above, the superimposing rate setting unit 101 has a configuration which includes an operation unit which can be operated by an image observer such as a doctor, for example, and can adjust a superimposing rate which is a ratio between a visible light image component and a fluorescence image component forming the superimposed image 30.

For example, a slide operation tab 121 as illustrated in Fig. 3 is included, and the image observer such as a doctor for observing the superimposed image 30 slides the slide operation tab 121.

By sliding the slide operation tab 121, it is possible to change the superimposing rates of the visible light image and the fluorescence image forming the superimposed image 30 displayed on the image display unit 120.

For example, when the slide operation tab 121 is set at the left end, an image including a visible light image of 100% is displayed on the image display unit 120.

When the slide operation tab 121 is moved to the right, the superimposing rate of the visible light image is gradually lowered, and the superimposing rate of the fluorescence image is increased.

Note that, in the image processing apparatus according to the present disclosure, in other words, the image processing apparatus 100 having the configuration illustrated in Fig. 2, the fluorescence image included in the superimposed image 30 is the noise reduced fluorescence image 112 generated by the fluorescence image noise reduction unit 105.

When the slide operation tab 121 is set at the center, the superimposing rate of the visible light image 10 coincides with the superimposing rate of the noise reduced fluorescence image 112, in other words, the superimposed image in which images are superimposed on one to one is displayed on the image display unit 120.

Moreover, when the slide operation tab 121 is moved to the right, the superimposing rate of the visible light image is gradually lowered, and the superimposing rate of the fluorescence image is increased. When the slide operation tab 121 is set at the right end, an image including only the noise reduced fluorescence image 112 is displayed on the image display unit 120.

Note that, in each embodiment to be described below, it is assumed that a superimposing rate $\alpha$ be a super-imposing ratio ($0 \leq \alpha \leq 1$) of the fluorescence image (noise reduced fluorescence image) in the superimposed image. A superimposed image having a superimposing rate of $\alpha = 0$ is a visible light image, and a superimposed image having a superimposing rate of $\alpha = 1$ is a fluorescence image (noise reduced fluorescence image).

A pixel value y of a pixel forming the superimposed image is indicated by the following formula (Formula 1).

$$y_{nr} = (1 - \alpha)y_V + \alpha y_{Fnr} \ldots (\text{Formula 1})$$

However, the reference $y_V$ indicates a pixel value of a visible light image, and
the reference $y_{Fnr}$ indicates a pixel value of a noise reduced fluorescence image.

Note that, the reference V is an index which means a visible light image, and the reference F is an index which means a fluorescence image.

The reference nr means a noise reduced image or a superimposed image to which the noise reduced image is applied.

Note that, in many cases, the visible light image to be superimposed is a color image, and the fluorescence image is a monotone black-and-white image.

To easily observe a fluorescence image region included in the superimposed image displayed on a display unit, pixel value correction for coloring the fluorescence image with a predetermined color such as green, for example, is performed before the superimposition processing, and the images may be superimposed after this correction.

In this way, the superimposing rate setting unit 101 of the image processing apparatus 100 has the configuration which includes the operation unit which can be operated by the image observer such as a doctor, for example, and can adjust the superimposing rate which is the ratio between the visible light image component and the fluorescence image component forming the superimposed image 30.

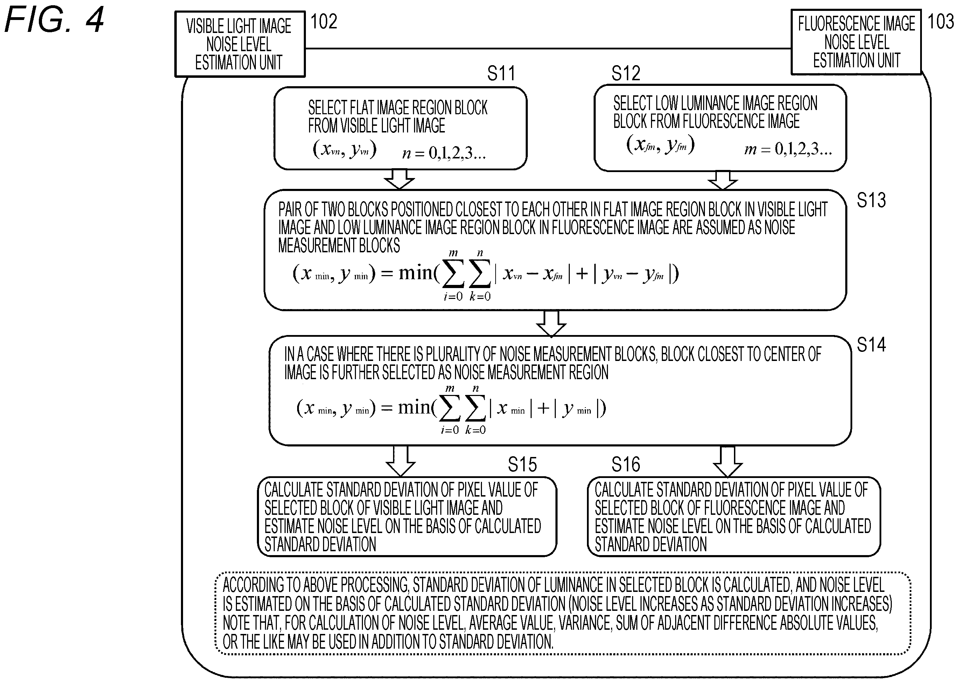

Next, specific processing executed by the visible light image noise level estimation unit 102 and the fluorescence image noise level estimation unit 103 will be described with reference to Fig. 4.

As described above, the visible light image noise level estimation unit 102 and the fluorescence image noise level estimation unit 103 respectively estimate noise levels included in the visible light image 10 and the fluorescence image 20.

Fig. 4 illustrates a sequence of the processing executed by the visible light image noise level estimation unit 102 and the fluorescence image noise level estimation unit 103.

Steps S11 to S14 are processing for selecting image regions (block) included in the visible light image 10 and the fluorescence image 20 for noise level estimation.

In other words, optimal image regions (block) for noise estimation, for example, rectangular blocks of which one side includes several-tens to several-hundreds pixels are selected from the visible light image 10 and the fluorescence image 20.

First, in step S11, a flat image region block is selected from the visible light image. In the visible light image, an image region having small pixel value change is a region where noise is easily detected, and the flat image region block is selected from the visible light image.

Here, the plurality of blocks below is selected. $(x_{vn}, y_{vn})$ n = 0, 1, 2, 3...

$(x_{vn}, y_{vn})$ is, for example, a center coordinate of the block.

On the other hand, in step S12, a low luminance image region block is selected from the fluorescence image. In the fluorescence image, the low luminance image region is a region where noise is easily detected, and the low luminance image region block is selected from the fluorescence image.

Here, the plurality of blocks below is selected. $(x_{fm}, y_{fm})$ m = 0, 1, 2, 3...

$(x_{fm}, y_{fm})$ is, for example, a center coordinate of the block.

Next, in step S13, a pair of two blocks closest to each other in the flat image region blocks in the visible light image selected in step S11 and the fluorescence image low luminance image region blocks selected in step S12 is assumed as noise measurement blocks.

The pair of two blocks to be selected according to (Formula 2) below is assumed as the noise measurement blocks.

[Equation 1]

$$(x_{\min}, y_{\min}) = \min\left(\sum_{i=0}^{m} \sum_{k=0}^{n} | x_{vn} - x_{fm} | + | y_{vn} - y_{fm} |\right)$$

$$\ldots \text{(Formula 2)}$$

In step S13, in a case where a single block pair cannot be selected, step S14 is executed.

In other words, in step S13, in a case where the plurality of noise measurement blocks is selected according to (Formula 2) described above, blocks closest to the center of the image is selected from among the plurality of block pairs in step S14.

In other words, the block pair closest to the center of the image selected according to (Formula 3) below is the noise measurement block.

[Equation 2]

$$(x_{\min}, y_{\min}) = \min\left(\sum_{i=0}^{m} \sum_{k=0}^{n} | x_{\min} | + | y_{\min} |\right)$$

$$\ldots \text{(Formula 3)}$$

After the noise measurement block has been determined by the processing in steps S11 to S14 described above, the noise levels of the visible light image and the fluorescence image are estimated on the basis of a pixel value distribution state of the selected block in next steps S15 and S16.

In step S15, a standard deviation of the pixel value of the selected block of the visible light image is calculated, and the noise level is estimated on the basis of the calculated standard deviation.

Furthermore, in step S16, a standard deviation of the pixel value of the selected block of the fluorescence image is calculated, and the noise level is estimated on the basis of the calculated standard deviation.

According to the processing above, the standard deviation of the pixel value (luminance) in the selected block is calculated, and the calculated standard deviation or a conversion value based on the standard deviation is assumed as a noise level.

The larger the standard deviation is, the larger the noise level is.

Note that, in addition to the standard deviation value, an average value of the pixel values (luminance), a variance, or a sum of adjacent difference absolute values, and the like of the pixels in the block may be used for the noise level calculation processing.

In this way, the visible light image noise level estimation unit 102 and the fluorescence image noise level estimation unit 103 respectively estimate the noise levels of noise included in the visible light image 10 and the fluorescence image 20.

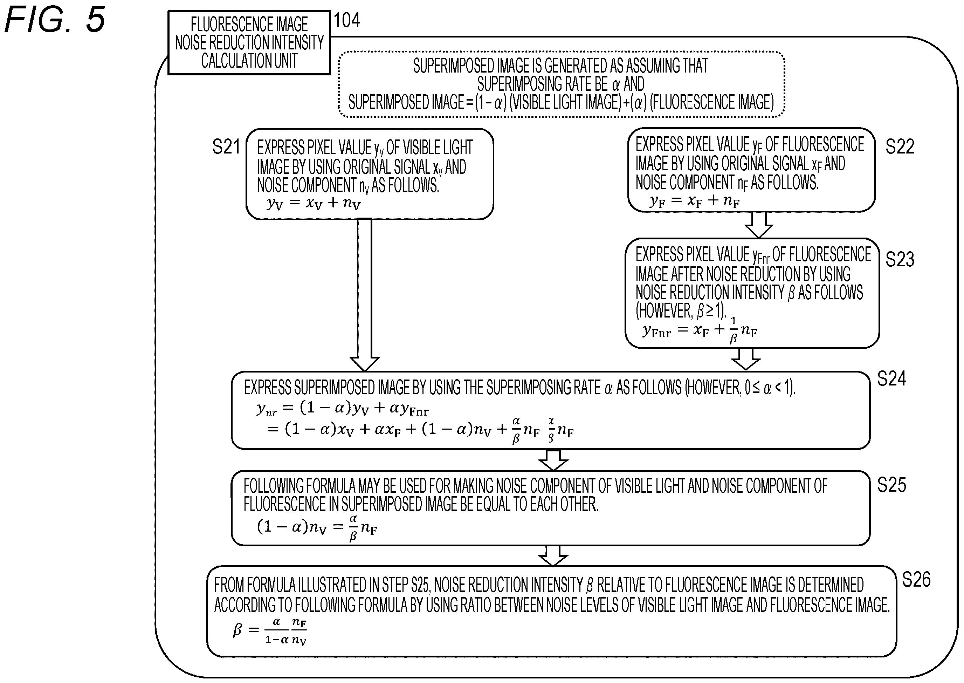

Next, processing executed by the fluorescence image noise reduction intensity calculation unit 104 of the image processing apparatus 100 illustrated in Fig. 2 will be described with reference to Fig. 5.

As described above with reference to Fig. 2, the fluorescence image noise reduction intensity calculation unit 104 calculates the noise level reduction intensity $\beta$, 111 which is a noise level reduction intensity of the fluorescence image 20 according to the noise levels of the visible light image 10 and the fluorescence image 20 respectively estimated by the visible light image noise level estimation unit 102 and the fluorescence image noise level estimation unit 103.

For example, even when the superimposing rates of the visible light image and the fluorescence image change, the fluorescence image noise reduction intensity calculation unit 104 calculates the noise level reduction intensity $\beta$, 111 according to the superimposing rate so that the noise levels of the visible light image and the fluorescence image included in the superimposed image are substantially coincide with each other.

Fig. 5 illustrates a processing sequence for calculating a noise level reduction intensity $\beta$ according to the superimposing rate executed by the fluorescence image noise reduction intensity calculation unit 104.

Note that, as a precondition, it is assumed that a superimposing rate be $\alpha$ (however, $0 \leq \alpha \leq 1$) and the superimposed image be generated by addition processing of an image to which the following superimposing rate is applied.

$$\text{Superimposed image} = (1 - \alpha) \ (\text{visible light image}) + (\alpha)$$

$$(\text{noise reduced fluorescence image})$$

Processing in steps S21 to S26 executed by the fluorescence image noise reduction intensity calculation unit 104 will be sequentially described below.

(Step S21)

First, in step S21, the fluorescence image noise reduction intensity calculation unit 104 expresses a pixel value $y_V$ of the visible light image by using an original signal $x_V$ and a noise component signal $n_V$ as the following formula.

$$y_V = x_V + n_V$$

Note that the original signal $x_V$ is an image signal which reflects a subject which does not include a noise component, and the noise component signal $n_V$ is a signal including only a noise component different from the image signal which reflects the subject.

(Step S22)

Next, in step S22, the fluorescence image noise reduction intensity calculation unit 104 expresses a pixel value $y_F$ of the fluorescence image by using an original signal $x_F$ and a noise component $n_F$ as the following formula.

$$y_F = x_F + n_F$$

(Step S23)

Next, in step S23, the fluorescence image noise reduction intensity calculation unit 104 expresses a pixel value $y_{Fnr}$ of the fluorescence image from which the noise has been reduced by using the original signal $x_F$, the noise reduction intensity $\beta$, and the noise component $n_F$ as the following formula.

$$y_{Fnr} = x_F + (1/\beta) n_F$$

Note that, as indicated in the above formula, by the noise reduction processing to which the noise reduction intensity $\beta$ is applied, the noise component included in the pixel value of the fluorescence image is decreased to $(1/\beta) n_F$.

(Step S24)

Next, in step S24, the fluorescence image noise reduction intensity calculation unit 104 expresses a superimposed image $y_{nr}$ of the visible light image $y_V$ and the noise reduced fluorescence image $y_{Fnr}$ by using the superimposing rate $\alpha$ as follows.

$$y_{nr} = (1 - \alpha)y_V + \alpha y_{Fnr}$$
$$= (1 - \alpha)x_V + \alpha x_F + (1 - \alpha)n_V + (\alpha/\beta)n_F$$

(Step S25)

Next, in step S25, the fluorescence image noise reduction intensity calculation unit 104 sets a formula which makes the noise component $n_V$ of the visible light image and the noise component $(\alpha/\beta)n_F$ of the noise reduced fluorescence image forming the superimposed image be equal to each other, in other words, the following formula (Formula 4).

$$(1 - \alpha)n_V = (\alpha/\beta)n_F ...(Formula\ 4)$$

(Formula 4) described above sets (a) a noise signal level of the visible light image $(1 - \alpha)n_V$ and (b) a noise signal level of the noise reduced fluorescence image $(\alpha/\beta)n_F$ included in the superimposed image to coincide with each other.

(Step S26)

Next, in step S26, the fluorescence image noise reduction intensity calculation unit 104 calculates the noise reduction intensity $\beta$ applied to the noise reduction processing executed on the fluorescence image according to the following (Formula 5), by using (Formula 4) described above.

$$\beta = (\alpha/(1 - \alpha))(n_F/n_V) ...(Formula\ 5)$$

In this way, the fluorescence image noise reduction intensity calculation unit 104 calculates the noise reduction intensity $\beta$ applied by the fluorescence image noise reduction unit 105 which executes the noise reduction processing on the fluorescence image 20.
As understood from (Formula 5) described above, the noise reduction intensity $\beta$ is set to increase as the superimposing rate $\alpha$ of the fluorescence image increases and decrease as the superimposing rate $\alpha$ of the fluorescence image decreases. The above (Formula 5) is derived from (Formula 4) described above, the above (Formula 4) sets (a) the noise signal level of the visible light image $(1 - \alpha)n_V$ and (b) the noise signal level of the noise reduced fluorescence image $(\alpha/\beta)n_F$ included in the superimposed image to coincide with each other.
In other words, by superimposing the noise reduced fluorescence image generated by executing the noise reduction processing to which the noise reduction intensity $\beta$ calculated according to (Formula 5) described above with the visible light image according to the superimposing rate $\alpha$, the noise signal level (intensity) of the visible light image and the noise signal level (intensity) of the noise reduced fluorescence image forming the superimposed image coincide with each other.
The noise signal levels change according to a change in the superimposing rate $\alpha$. However, even when the superimposing rate $\alpha$ changes, the noise signal level (intensity) of the visible light image and the noise signal level (intensity) of the noise reduced fluorescence image forming the superimposed image are controlled to be set so that the noise signal levels substantially coincide with each other.
As understood from (Formula 5) described above, in a case where the superimposing rate $\alpha$ increases, in other words, the superimposing rate of the noise reduced fluorescence image increases, the noise reduction intensity $\beta$ increases, and the fluorescence image noise reduction unit 105 executes processing for reducing the noise of the fluorescence image 20.
On the other hand, in a case where the superimposing rate $\alpha$ is decreased, in other words, the superimposing rate of the noise reduced fluorescence image is decreased, the noise reduction intensity $\beta$ is decreased, and the fluorescence image noise reduction unit 105 weakens the noise reduction intensity to be applied and executes the noise reduction processing on the fluorescence image 20.

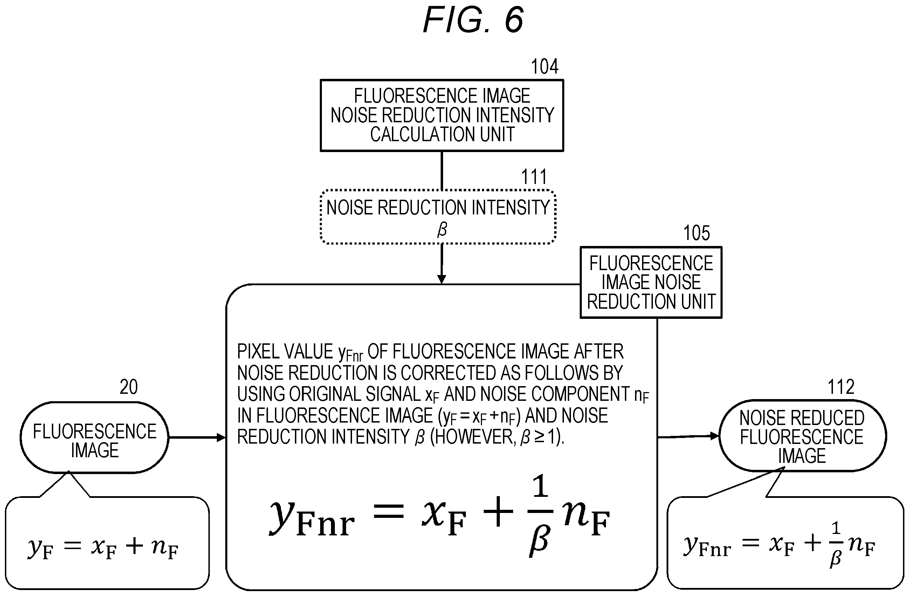

Next, a specific example of processing executed by the fluorescence image noise reduction unit 105 will be described with reference to Fig. 6.

The fluorescence image noise reduction unit 105 executes noise level reduction processing on the fluorescence image 20 according to the noise level reduction intensity $\beta$, 111 calculated by the fluorescence image noise reduction intensity calculation unit 104.

As illustrated in Fig. 6, the fluorescence image noise reduction unit 105 receives the fluorescence image 20 and further receives the noise level reduction intensity $\beta$, 111 calculated by the fluorescence image noise reduction intensity calculation unit 104.

The pixel value $y_F$ of the fluorescence image 20 is indicated as follows by using the original signal $x_F$ and the noise component $n_F$ as described above in step S22 in Fig. 5.

$$y_F = x_F + n_F$$

Note that the original signal $x_V$ is an image signal which reflects a subject which does not include a noise component, and the noise component signal $n_V$ is a signal including only a noise component different from the image signal which reflects the subject.

The fluorescence image noise reduction unit 105 applies the noise level reduction intensity $\beta$ calculated by the fluorescence image noise reduction intensity calculation unit 104 and generates the noise reduced fluorescence image 112 having the pixel value $y_{Fnr}$ from which the noise has been reduced according to the following (Formula 6).

$$y_{Fnr} = x_F + (1/\beta)n_F...(Formula\ 6)$$

As indicated in the above formula, by the noise reduction processing to which the noise reduction intensity $\beta$ is applied, the noise component included in the pixel value of the fluorescence image is decreased to $(1/\beta)n_F$.

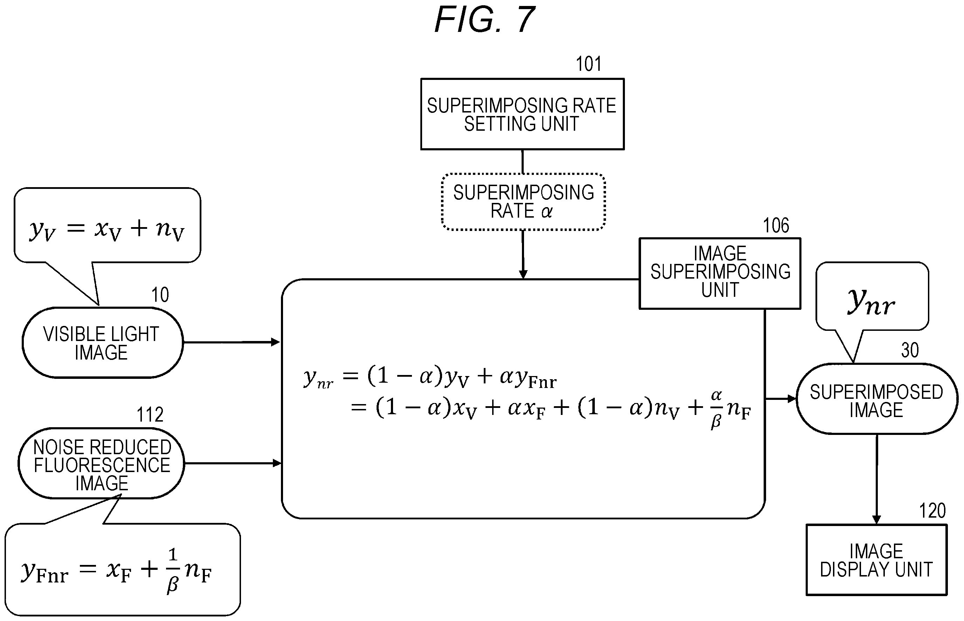

Next, processing executed by the image superimposing unit 106 will be described with reference to Fig. 7.

The image superimposing unit 106 superimposes the visible light image 10 with a noise reduced fluorescence image 112 generated by the fluorescence image noise reduction unit 105 and generates the superimposed image 30.

The generated superimposed image 30 is displayed on the image display unit 120.

As illustrated in Fig. 7, the image superimposing unit 106 receives the visible light image 10 and the noise reduced fluorescence image 112 generated by the fluorescence image noise reduction unit 105.

Moreover, the superimposing rate setting unit 101 inputs the superimposing rate $\alpha$ set by, for example, an image observer such as a doctor.

The pixel value $y_V$ of the visible light image 10 is expressed by the following formula as illustrated in Fig. 7.

$$y_V = x_V + n_V$$

Furthermore, as illustrated in Fig. 7, the pixel value $y_{Fnr}$ of the noise level reduction fluorescence image 112 generated by the fluorescence image noise reduction unit 105 is expressed by the following formula.

$$y_{Fnr} = x_F + (1/\beta)n_F$$

The image superimposing unit 106 superimposes the two images according to the superimposing rate $\alpha$ and generates the superimposed image 30. The pixel value $y_{nr}$ of the superimposed image 30 is expressed by the following formula (Formula 7).

$$y_{nr} = (1 - \alpha)y_V + \alpha y_{Fnr}$$
$$= (1 - \alpha)x_V + \alpha x_F + (1 - \alpha)n_V + (\alpha/\beta)n_F...(Formula\ 7)$$

In this way, the image superimposing unit 106 superimposes the visible light image 10 with the noise reduced fluorescence image 112 generated by the fluorescence image noise reduction unit 105 and generates the superimposed image 30, and the generated superimposed image 30 is output to and displayed on the image display unit 120.

As understood from (Formula 5) described above, the noise reduction intensity $\beta$ indicated in (Formula 7) described above is set to increase as the superimposing rate $\alpha$ of the fluorescence image increases and decrease as the superimposing rate $\alpha$ of the fluorescence image decreases.

(Formula 5) described above is derived from (Formula 4) described above, (Formula 4) described above sets (a) the noise signal level of the visible light image $(1 - \alpha)n_V$ and (b) the noise signal level of the noise reduced fluorescence image $(\alpha/\beta)n_F$ included in the superimposed image to coincide with each other.

In other words, by superimposing the noise reduced fluorescence image generated by executing the noise reduction processing to which the noise reduction intensity $\beta$ calculated according to (Formula 5) described above with the visible light image according to the superimposing rate $\alpha$, the noise signal level (intensity) of the visible light image and the noise signal level (intensity) of the noise reduced fluorescence image forming the superimposed image coincide with each other.

In other words, the noise signal level (intensity) of the visible light image included in the superimposed image calculated according to (Formula 7) described above and the noise signal level (intensity) of the noise reduced fluorescence image are levels (intensity) substantially coincide with each other.

The noise signal levels change according to a change in the superimposing rate $\alpha$. However, even when the superimposing rate $\alpha$ changes, the noise signal level (intensity) of the visible light image and the noise signal level (intensity) of the noise reduced fluorescence image forming the superimposed image are controlled to be set so that the noise signal levels substantially coincide with each other.

In a case where the superimposing rate $\alpha$ increases, in other words, the superimposing rate of the noise reduced fluorescence image increases, the noise reduction intensity $\beta$ increases, and the fluorescence image noise reduction unit 105 executes processing for largely reducing the noise of the fluorescence image 20.

As a result, the noise level of the noise reduced fluorescence image 112 included in the superimposed image indicated by (Formula 7) described above is set to substantially coincide with the noise level of the visible light image 20 included in the superimposed image.

On the other hand, in a case where the superimposing rate $\alpha$ is decreased, in other words, the superimposing rate of the noise reduced fluorescence image is decreased, the noise reduction intensity $\beta$ is decreased, and the fluorescence image noise reduction unit 105 weakens the noise reduction intensity to be applied and executes the noise reduction processing on the fluorescence image 20.

As a result, the noise level of the noise reduced fluorescence image 112 included in the superimposed image indicated by (Formula 7) described above is set to substantially coincide with the noise level of the visible light image 20 included in the superimposed image.

In this way, even when the superimposing rate $\alpha$ changes, it is possible to generate and display the superimposed image which is controlled to have setting such that the noise signal level (intensity) of the visible light image and the noise signal level (intensity) of the noise reduced fluorescence image forming the superimposed image substantially coincide with each other.

In other words, setting is controlled so that the noise level of the noise reduced fluorescence image constantly coincides with the noise level of the visible light image, and the superimposed image is displayed. Therefore, even when the superimposing rate of the superimposed image is changed and a ratio of the fluorescence image is changed, the increase or decrease of the noise is suppressed, and an image which can be easily observed can be displayed.

[3. Sequence of processing executed by image processing apparatus]

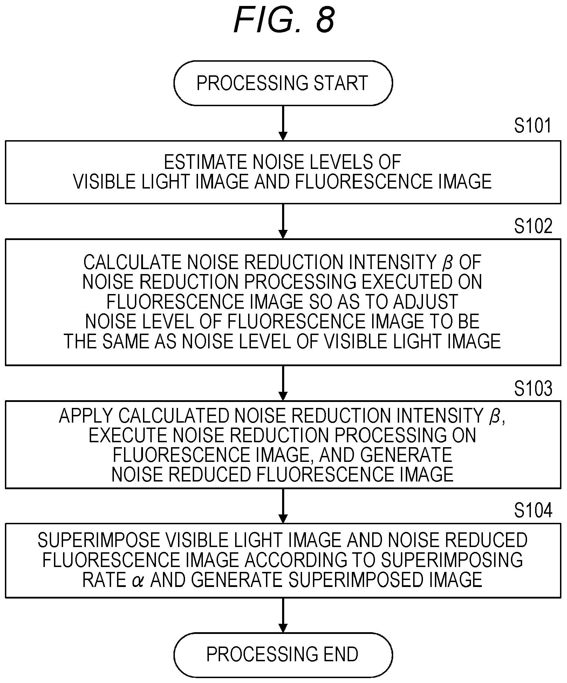

Next, a sequence of processing executed by the image processing apparatus 100 illustrated in Fig. 2 will be described with reference to the flowchart in Fig. 8.

The processing according to the flow illustrated in Fig. 8 is executed, for example, under control of a control unit having a program execution function according to a program stored in a storage unit of the image processing apparatus.

Processing in each step of the flow illustrated in Fig. 8 will be sequentially described below.

(Step S101)

First, the image processing apparatus estimates noise levels of the visible light image and the fluorescence image in step S101.

This processing is executed by the visible light image noise level estimation unit 102 and the fluorescence image noise level estimation unit 103 of the image processing apparatus 100 illustrated in Fig. 2.

The visible light image noise level estimation unit 102 and the fluorescence image noise level estimation unit 103 respectively estimate noise levels of noise included in the visible light image 10 and the fluorescence image 20.

As described above with reference to Fig. 4, regarding the pixel blocks set in the visible light image 10 and the fluorescence image 20, the blocks are selected from a flat region of the visible light image 10 and selected from a low luminance region of the fluorescence image 20, a block pair of which the blocks are close to each other and close to the center of the image is selected. Then, this selected block pair is set as a pixel block applied to the noise level estimation processing.

Regarding the block of the visible light image and the block of the fluorescence image selected as the pixel blocks applied to the noise level estimation processing, for example, the standard deviation of the pixel value (luminance) is calculated, and the calculated value is assumed as a noise level.

Note that, in addition to the standard deviation value, an average value of the pixel values (luminance), a variance, or a sum of adjacent difference absolute values, and the like of the pixels in the block may be used.

(Step S102)

Next, the image processing apparatus calculates the noise reduction intensity $\beta$ of the noise reduction processing executed on the fluorescence image so as to adjust the noise level of the fluorescence image to be the same as the noise level of the visible light image in step S102.

This processing is executed by the fluorescence image noise reduction intensity calculation unit 104 of the image processing apparatus 100 illustrated in Fig. 2.

As described above with reference to Fig. 5, the fluorescence image noise reduction intensity calculation unit 104 executes the processing in steps S21 to S26 illustrated in Fig. 5 and calculates the noise level reduction intensity $\beta$ according to the superimposing rate so that the noise levels of the visible light image and the fluorescence image included in the superimposed image substantially coincide with each other even when the superimposing rate $\alpha$ of the visible light image and the fluorescence image changes.

The noise reduction intensity $\beta$ is calculated according to (Formula 5) below as described above.

$$\beta \ = \ (\alpha/(1 \ - \ \alpha))(n_F/n_V)...(\text{Formula 5})$$

As understood from (Formula 5) described above, the noise reduction intensity $\beta$ is set to increase as the superimposing rate $\alpha$ of the fluorescence image increases and decrease as the superimposing rate $\alpha$ of the fluorescence image decreases.

(Step S103)

Next, in step S103, the image processing apparatus applies the calculated noise reduction intensity $\beta$, executes the noise reduction processing on the fluorescence image, and generates a noise reduced fluorescence image.

This processing is executed by the fluorescence image noise reduction unit 105 of the image processing apparatus 100 illustrated in Fig. 2.

As described above with reference to Fig. 6, the fluorescence image noise reduction unit 105 applies the noise level reduction intensity $\beta$ calculated by the fluorescence image noise reduction intensity calculation unit 104 and generates the noise reduced fluorescence image 112 having the pixel value $y_{Fnr}$ from which the noise has been reduced according to the following (Formula 6).

$$y_{Fnr} \ = \ x_F \ + \ (1/\beta)n_F...(\text{Formula 6})$$

As indicated in the above formula, by the noise reduction processing to which the noise reduction intensity $\beta$ is applied, the noise component included in the pixel value of the fluorescence image is decreased to $(1/\beta)n_F$.

(Step S104)

Next, in step S104, the image processing apparatus superimposes the visible light image with the noise reduced fluorescence image according to the superimposing rate $\alpha$ and generates the superimposed image.

This processing is executed by the image superimposing unit 106 of the image processing apparatus 100 illustrated in Fig. 2.

As described above with reference to Fig. 7, the image superimposing unit 106 superimposes the visible light image 10 with the noise reduced fluorescence image 112 generated by the fluorescence image noise reduction unit 105 and generates the superimposed image 30.

The generated superimposed image 30 is displayed on the image display unit 120.

As described above with reference to Fig. 7, the image superimposing unit 106 superimposes the visible light image 10 with the noise reduced fluorescence image 112 generated by the fluorescence image noise reduction unit 105 according to the superimposing rate $\alpha$ and generates the superimposed image 30. The pixel value $y_{nr}$ of the superimposed image 30 is expressed by the following formula (Formula 7).

$$y_{nr} = (1 - \alpha) y_V + \alpha y_{Fnr}$$
$$= (1 - \alpha) x_V + \alpha x_F + (1 - \alpha) n_V + (\alpha/\beta) n_F \ldots (\text{Formula 7})$$

In this way, the image superimposing unit 106 superimposes the visible light image 10 with the noise reduced fluorescence image 112 generated by the fluorescence image noise reduction unit 105 and generates the superimposed image 30, and the generated superimposed image 30 is output to and displayed on the image display unit 120.

As understood from (Formula 5) described above, the noise reduction intensity $\beta$ indicated in (Formula 7) described above is set to increase as the superimposing rate $\alpha$ of the fluorescence image increases and decrease as the superimposing rate $\alpha$ of the fluorescence image decreases.

(Formula 5) described above is derived from (Formula 4) described above, (Formula 4) described above sets (a) the noise signal level of the visible light image $(1 - \alpha) n_V$ and (b) the noise signal level of the noise reduced fluorescence image $(\alpha/\beta) n_F$ included in the superimposed image to coincide with each other.

In other words, by superimposing the noise reduced fluorescence image generated by executing the noise reduction processing to which the noise reduction intensity $\beta$ calculated according to (Formula 5) described above with the visible light image according to the superimposing rate $\alpha$, the noise signal level (intensity) of the visible light image and the noise signal level (intensity) of the noise reduced fluorescence image forming the superimposed image coincide with each other.

In other words, the noise signal level (intensity) of the visible light image included in the superimposed image calculated according to (Formula 7) described above and the noise signal level (intensity) of the noise reduced fluorescence image are levels (intensity) substantially coincide with each other.

The noise signal levels change according to a change in the superimposing rate $\alpha$. However, even when the superimposing rate $\alpha$ changes, the noise signal level (intensity) of the visible light image and the noise signal level (intensity) of the noise reduced fluorescence image forming the superimposed image are controlled to be set so that the noise signal levels substantially coincide with each other.

In a case where the superimposing rate $\alpha$ increases, in other words, the superimposing rate of the noise reduced fluorescence image increases, the noise reduction intensity $\beta$ increases, and the fluorescence image noise reduction unit 105 executes processing for largely reducing the noise of the fluorescence image 20.

As a result, the noise level of the noise reduced fluorescence image 112 included in the superimposed image indicated by (Formula 7) described above is set to substantially coincide with the noise level of the visible light image 20 included in the superimposed image.

On the other hand, in a case where the superimposing rate $\alpha$ is decreased, in other words, the superimposing rate of the noise reduced fluorescence image is decreased, the noise reduction intensity $\beta$ is decreased, and the fluorescence image noise reduction unit 105 weakens the noise reduction intensity to be applied and executes the noise reduction processing on the fluorescence image 20.

As a result, the noise level of the noise reduced fluorescence image 112 included in the superimposed image indicated by (Formula 7) described above is set to substantially coincide with the noise level of the visible light image 20 included in the superimposed image.

In this way, even when the superimposing rate $\alpha$ changes, it is possible to generate and display the superimposed image which is controlled to have setting such that the noise signal level (intensity) of the visible light image and the noise signal level (intensity) of the noise reduced fluorescence image forming the superimposed image substantially coincide with each other.

In other words, setting is controlled so that the noise level of the noise reduced fluorescence image constantly coincides with the noise level of the visible light image, and the superimposed image is displayed. Therefore, even when the superimposing rate of the superimposed image is changed and a ratio of the fluorescence image is changed, the increase or decrease of the noise is suppressed, and an image which can be easily observed can be displayed.

[4. Configuration and processing of second embodiment of image processing apparatus according to present disclosure]

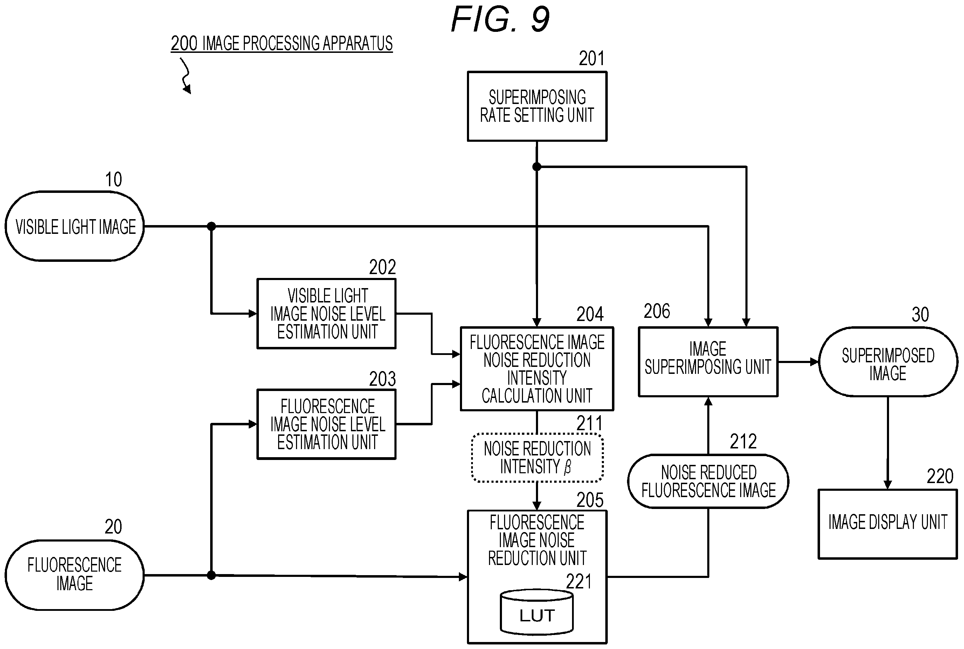

Next, a configuration and processing of a second embodiment of the image processing apparatus according to the present disclosure will be described with reference to Fig. 9 and subsequent drawings.

[4-1. Exemplary configuration for determining filter size on the basis of noise reduction intensity]

First, an exemplary configuration for determining a filter size on the basis of a noise reduction intensity will be described.

Fig. 9 is a block diagram illustrating an exemplary configuration of an image processing apparatus 200 according to the second embodiment of the present disclosure.

Similarly to the image processing apparatus 100 illustrated in Fig. 2 described as the first embodiment above, the image processing apparatus 200 according to the second embodiment illustrated in Fig. 9 receives a visible light image 10 and a fluorescence image 20 and generates a superimposed image 30 obtained by superimposing the two images and outputs the superimposed image 30. The superimposed image 30 is, for example, output to and displayed by an image display device 220 having an image display unit (display).

Similarly to the image processing apparatus 100 illustrated in Fig. 2 described as the first embodiment above, the image processing apparatus 200 illustrated in Fig. 9 has a configuration for controlling a noise level of the superimposed image 30 obtained by superimposing the visible light image 10 and the fluorescence image 20. Specifically, for example, a configuration is included which can lower the noise level of the fluorescence image 20 to the noise level of the visible light image 10 which is a superimposing target and superimposes the fluorescence image 20 and the visible light image 10.

As illustrated in Fig. 9, the image processing apparatus 200 includes a superimposing rate setting unit 201, a visible light image noise level estimation unit 202, a fluorescence image noise level estimation unit 203, a fluorescence image noise reduction intensity calculation unit 204, a fluorescence image noise reduction unit 205, and an image superimposing unit 206.

These components have the configurations similar to those of the components of the image processing apparatus 100 illustrated in Fig. 2 described as the first embodiment above.

A difference between the image processing apparatus 200 illustrated in Fig. 9 and the image processing apparatus 100 illustrated in Fig. 2 is the fluorescence image noise reduction unit 205. The fluorescence image noise reduction unit 205 of the image processing apparatus 200 illustrated in Fig. 9 includes a look-up table (LUT) 221 as illustrated in Fig. 9.

The fluorescence image noise reduction unit 205 refers to data stored in the look-up table (LUT) 221 and executes noise level reduction processing on the fluorescence image 20.

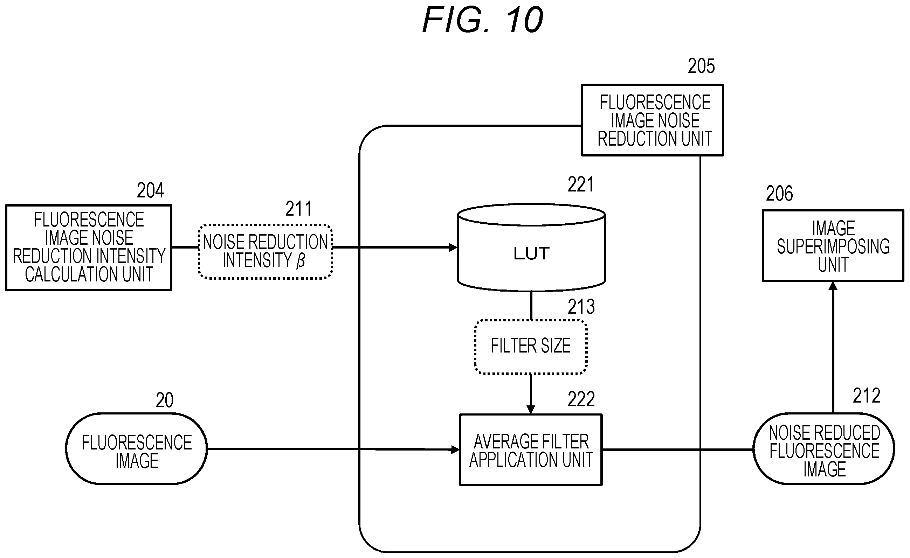

A specific configuration and processing of the fluorescence image noise reduction unit 205 will be described with reference to Fig. 10.

As illustrated in Fig. 10, the fluorescence image noise reduction unit 205 includes the look-up table (LUT) 221 and an average filter application unit 222.

As illustrated in Fig. 10, the fluorescence image noise reduction unit 205 receives the fluorescence image 20 and further receives a noise level reduction intensity $\beta$, 211 calculated by the fluorescence image noise reduction intensity calculation unit 204.

The look-up table (LUT) 221 stores data in which the noise reduction intensity $\beta$ is associated with an average filter size optimal for the noise reduction intensity $\beta$.

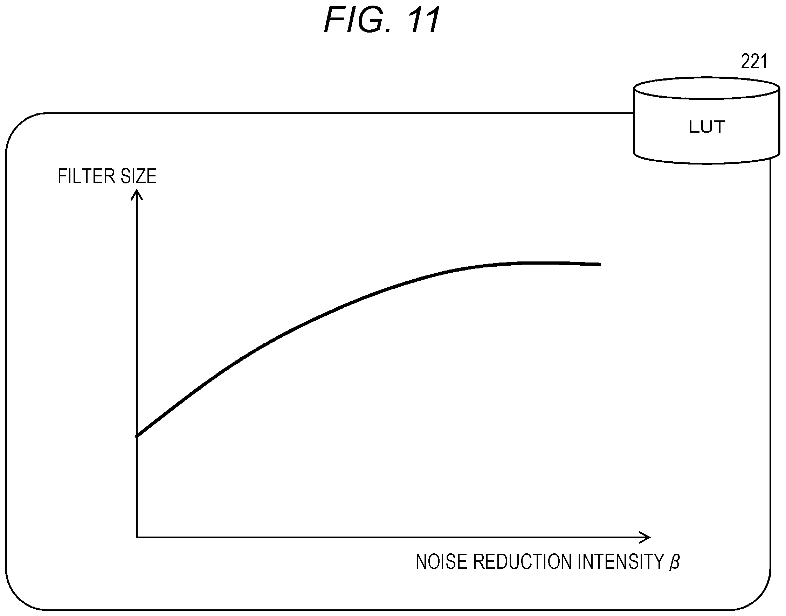

An example of the data stored in the look-up table (LUT) 221 is illustrated in Fig. 11.

In Fig. 11, a graph is illustrated in which the noise reduction intensity $\beta$ is set on the horizontal axis and the average filter size is set on the vertical axis. A line illustrated in the graph is data indicating the optimal average filter size associated with the values of various noise reduction intensities $\beta$.

In the look-up table (LUT) 221, such association data is stored in advance, and the fluorescence image noise reduction unit 205 acquires the optimal average filter size on the basis of the noise level reduction intensity $\beta$, 211 input from the fluorescence image noise reduction intensity calculation unit 204 and outputs an acquired filter size 213 to the average filter application unit 222.

The average filter application unit 222 applies an average filter having a size determined by the filter size 213 input from the LUT 221 to the fluorescence image 20 and executes the noise reduction processing on the fluorescence image.

A noise reduced fluorescence image 212 is generated according to this average filter application processing.

Note that the average filter is a filter which calculates an average pixel value of all the pixels in a pixel region centered on a pixel to be corrected, for example, a pixel region including $n \times n$ pixels and sets the calculated pixel value as a correction pixel value. For example, the reference n is several pixels to several tens pixels.

As understood from the graph (LUT) in Fig. 11, as the noise level reduction intensity $\beta$ input from the fluorescence image noise reduction intensity calculation unit 204 increases, the filter size is set to be larger.

In other words, as the noise level reduction intensity $\beta$ is larger, averaging processing using pixels in a wider region is executed. That is, an noise reduction effect is enhanced.

In the second embodiment, a table in which the noise level reduction intensity β is associated with the average filter size is stored as the look-up table (LUT) 221 in advance, and an average filter having a size selected on the basis of the table data is applied to the fluorescence image 20 so as to generate the noise reduced fluorescence image 212.

Note that, in the embodiment described above, a configuration in which the average filter is applied when the noise reduced fluorescence image 212 is generated from the fluorescence image 20 has been described. However, this is an example, and the filter applied when the noise reduced fluorescence image 212 is generated from the fluorescence image 20 is not limited to the average filter, and various filters such as a median filter and a bilateral filter can be applied.

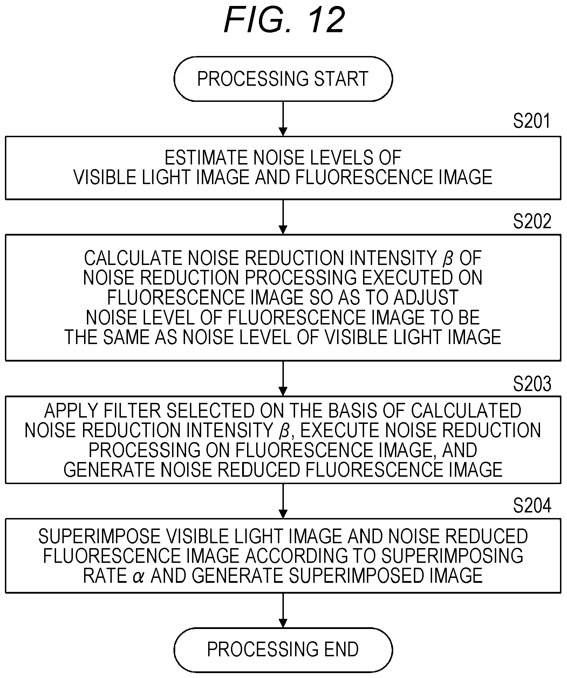

Next, a sequence of processing executed by the image processing apparatus 200 illustrated in Fig. 9 according to the present embodiment will be described with reference to the flowchart in Fig. 12.

Processing in each step of the flow illustrated in Fig. 12 will be sequentially described below.

(Steps S201 and S202)

Processing in steps S201 and S202 is similar to the processing in steps S101 and S102 in the flowchart illustrated in Fig. 8 which is the processing according to the first embodiment described above.

First, the image processing apparatus estimates noise levels of the visible light image and the fluorescence image in step S201.

This processing is executed by the visible light image noise level estimation unit 202 and the fluorescence image noise level estimation unit 203 of the image processing apparatus 200 illustrated in Fig. 9.

The visible light image noise level estimation unit 202 and the fluorescence image noise level estimation unit 203 respectively estimate noise levels included in the visible light image 10 and the fluorescence image 20 according to the sequence described with reference to Fig. 4 above.

Next, the image processing apparatus calculates the noise reduction intensity β of the noise reduction processing executed on the fluorescence image so as to adjust the noise level of the fluorescence image to be the same as the noise level of the visible light image in step S202.

This processing is executed by the fluorescence image noise reduction intensity calculation unit 204 of the image processing apparatus 200 illustrated in Fig. 9.

As described above with reference to Fig. 5, the fluorescence image noise reduction intensity calculation unit 204 executes the processing in steps S21 to S26 illustrated in Fig. 5 and calculates the noise level reduction intensity β according to a superimposing rate so that the noise levels of the visible light image and the noise reduced fluorescence image included in the superimposed image are substantially the same even when the superimposing rate α of the visible light image and the noise reduced fluorescence image changes.

(Step S203)

Next, in step S203, the image processing apparatus applies the calculated noise reduction intensity β, executes the noise reduction processing on the fluorescence image, and generates a noise reduced fluorescence image.

This processing is executed by the fluorescence image noise reduction unit 205 of the image processing apparatus 200 illustrated in Fig. 9.

As described above with reference to Fig. 10, the fluorescence image noise reduction unit 205 receives the noise level reduction intensity β calculated by the fluorescence image noise reduction intensity calculation unit 204 and acquires the optimal average filter size associated with the value of the noise reduction intensity β from the look-up table (LUT) 221.

Moreover, the acquired filter size 213 is output to the average filter application unit 222.

The average filter application unit 222 applies an average filter having a size determined by the filter size 213 input from the LUT 221 to the fluorescence image 20 and executes the noise reduction processing on the fluorescence image.

A noise reduced fluorescence image 212 is generated according to this average filter application processing.

(Step S204)

Next, in step S204, the image processing apparatus superimposes the visible light image with the noise reduced fluorescence image according to the superimposing rate α and generates the superimposed image.

This processing is executed by the image superimposing unit 206 of the image processing apparatus 200 illustrated in Fig. 9.

This processing is similar to the processing described with reference to Fig. 7 in the first embodiment above. The image superimposing unit 206 superimposes the visible light image 10 with the noise reduced fluorescence image 212 generated by the fluorescence image noise reduction unit 205 and generates the superimposed image 30.

The generated superimposed image 30 is displayed on the image display unit 220.

As described above with reference to Fig. 7, the image superimposing unit 106 superimposes the visible light image 10 with the noise reduced fluorescence image 112 generated by the fluorescence image noise reduction unit 105 according to the superimposing rate $\alpha$ and generates the superimposed image 30. A pixel value $y_{nr}$ of the superimposed image 30 is indicated by the formula (Formula 7) described above.

In the present embodiment, the noise signal level of the superimposed image changes according to a change in the superimposing rate $\alpha$. However, even when the superimposing rate $\alpha$ changes, the noise signal level (intensity) of the visible light image and the noise signal level (intensity) of the noise reduced fluorescence image forming the superimposed image are controlled to be set so that the noise signal levels substantially coincide with each other.

In other words, setting is controlled so that the noise level of the noise reduced fluorescence image constantly coincides with the noise level of the visible light image, and the superimposed image is displayed. Therefore, even when the superimposing rate of the superimposed image is changed and a ratio of the fluorescence image is changed, the increase or decrease of the noise is suppressed, and an image which can be easily observed can be displayed.

[4-2. Exemplary configuration for determining filter size on the basis of learning data based on noise reduction intensity and image feature amount]

Next, an exemplary configuration for determining a filter size on the basis of learning data based on a noise reduction intensity and an image feature amount will be described as a modification of the second embodiment.

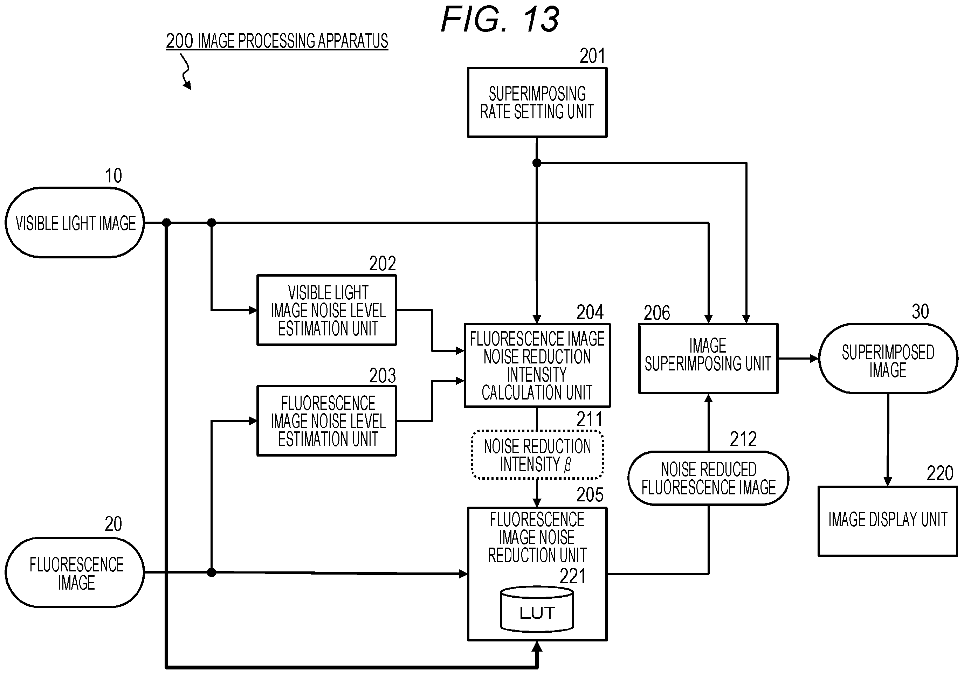

Fig. 13 is a block diagram illustrating an exemplary configuration of the image processing apparatus 200 according to the modification of the second embodiment of the present disclosure.

The image processing apparatus 200 illustrated in Fig. 13 has a configuration substantially similar to the configuration of the image processing apparatus 200 described above with reference to Fig. 9, the fluorescence image noise reduction unit 205 includes the look-up table (LUT) 221.

A difference from the configuration of the image processing apparatus 200 illustrated in Fig. 9 is that the fluorescence image noise reduction unit 205 receives the visible light image 10 together with the fluorescence image 20.

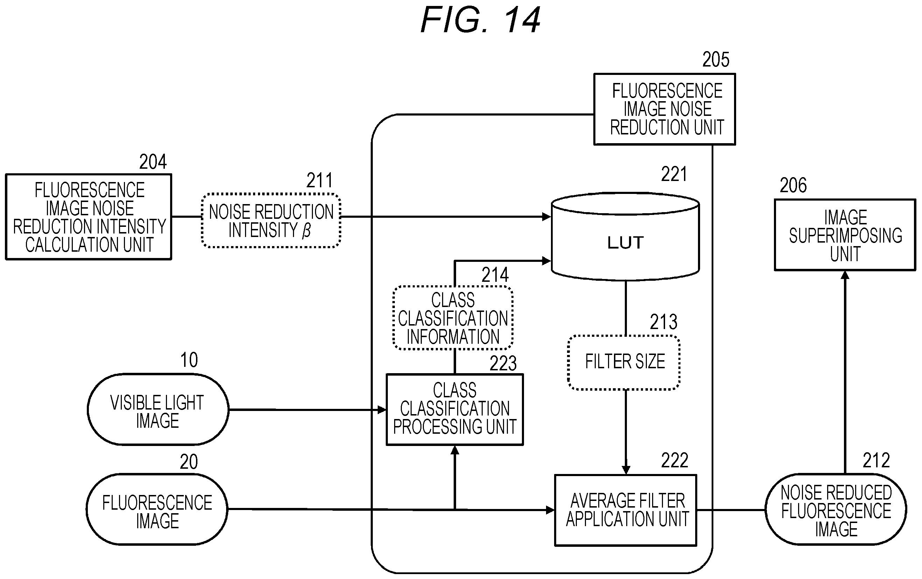

A specific configuration and processing of the fluorescence image noise reduction unit 205 will be described with reference to Fig. 14.

As illustrated in Fig. 14, similarly to the fluorescence image noise reduction unit 205 described above with reference to Fig. 10, the fluorescence image noise reduction unit 205 includes the look-up table (LUT) 221 and the average filter application unit 222.

The fluorescence image noise reduction unit 205 illustrated in Fig. 14 further includes a class classification processing unit 223.

The class classification processing unit 223 receives the visible light image 10 and the fluorescence image 20 and outputs class classification information 214 by applying learning data, which has been acquired in advance, on the basis of the feature amounts of the two images.

The look-up table (LUT) 221 stores the noise reduction intensity $\beta$, 211 and an optimal average filter size associated with the class classification information 214.

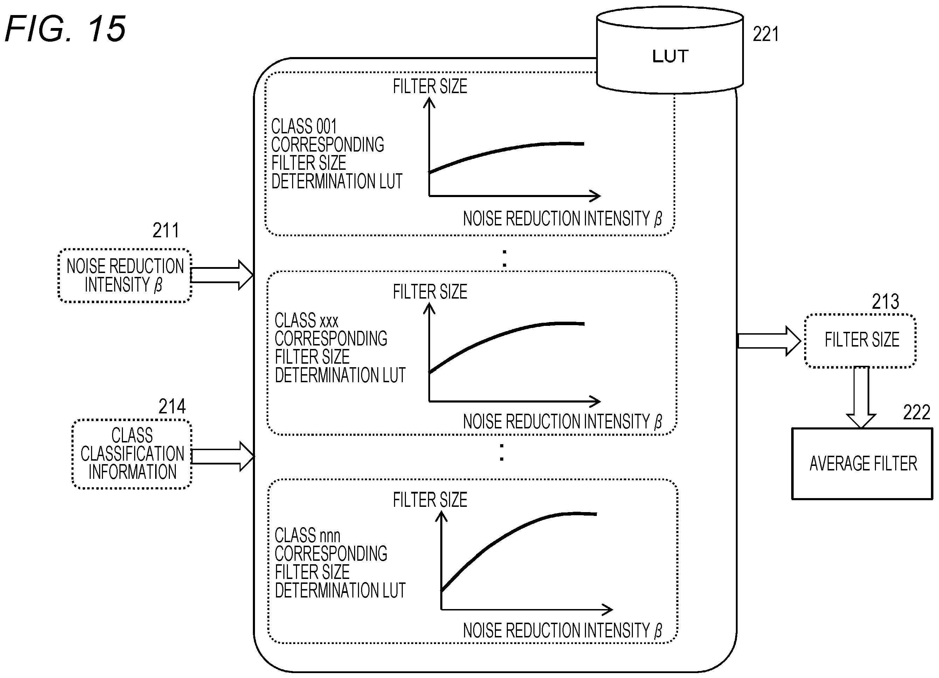

An example of the data stored in the look-up table (LUT) 221 is illustrated in Fig. 15.

In Fig. 15, a plurality of graphs (000 to nnn) is illustrated in which the noise reduction intensity $\beta$ is set on the horizontal axis and the average filter size is set on the vertical axis. A line illustrated in each graph is data indicating the optimal average filter size associated with the values of various noise reduction intensities $\beta$.

Each of the plurality of graphs (LUT) illustrated in Fig. 15 is a graph (LUT) corresponding to a class classified by applying the learning acquired in advance on the basis of the feature amounts of the two images including the visible light image 10 and the fluorescence image 20.

In the LUT 221, data corresponding to the class classification information 214 (LUT indicated in graph) is selected, and a filter size associated with the input noise reduction intensity $\beta$, 211 is acquired from the selected data, and the acquired filter size 213 is output to the average filter application unit 222.

The average filter application unit 222 applies an average filter having a size determined by the filter size 213 input from the LUT 221 to the fluorescence image 20 and executes the noise reduction processing on the fluorescence image.

A noise reduced fluorescence image 212 is generated according to this average filter application processing.

Note that, as described above, the class classification processing unit 223 receives the visible light image 10 and the fluorescence image 20 and outputs the class classification information 214 by applying the learning data, which has been acquired in advance, on the basis of the feature amounts of the two images.

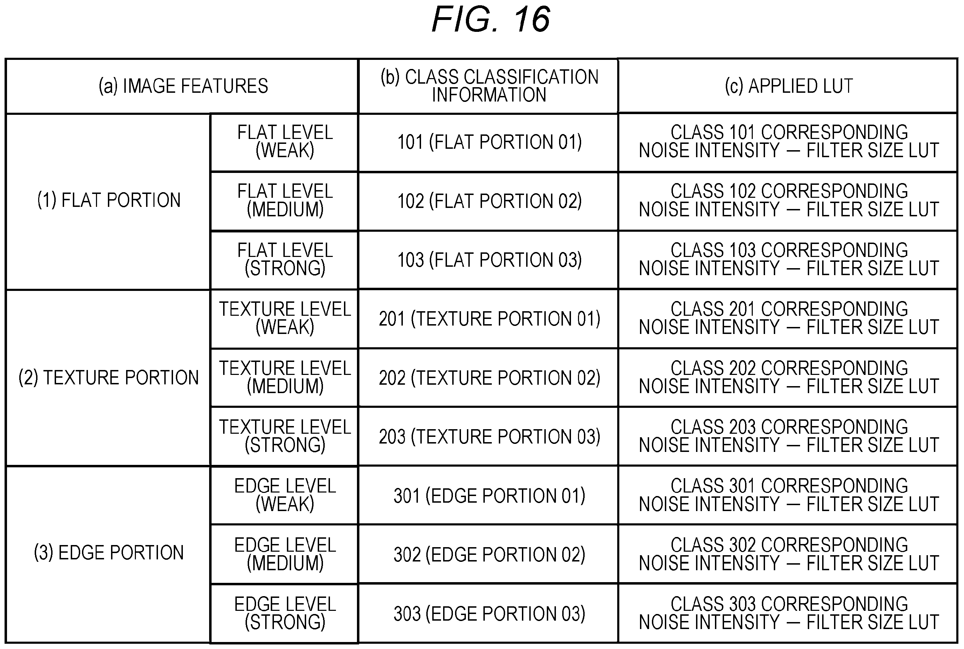

An output example of the class classification information 214 by the class classification processing unit 223 will be described with reference to Fig. 16.

In Fig. 16,

(a) Image feature
(b) Class classification information
(c) Applied LUT

these corresponding data is illustrated.

(a) The image features correspond to the feature amounts of the two images including the visible light image 10 and the fluorescence image 20 to be input.
Specifically, a flat portion, a texture portion, an edge portion, and the like are distinguished from each other according to the image feature amounts. Moreover, each of the distinguished image feature is divided into strong, medium, and weak levels.
(b) The class classification information is a class identifier associated with the image feature amount set by the prior learning processing.
(c) The applied LUT is an identifier of the graph (LUT) corresponding to the class described with reference to Fig. 15.

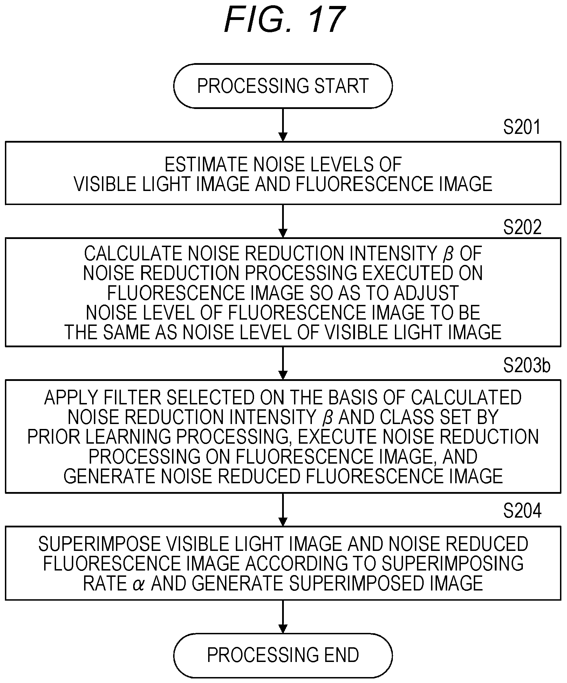

The class classification processing unit 223 receives the visible light image 10 and the fluorescence image 20 and acquires the feature amounts of the two images. This feature amounts correspond to (a) image features.
Next, the class classification information 214 is acquired on the basis of (a) image features.
(a) Image features and (b) class classification information illustrated in Fig. 16 are associated with each other by applying the learning data to which a sample image imaged in advance is applied, and the class classification information 214 associated with the image feature amounts of the received visible light image 10 and fluorescence image 20 is selected and output by using the associated data.
Thereafter, according to the class classification information 214, a piece of data, in other words, one of the plurality of graphs illustrated in Fig. 15 is selected from among the LUTs 221 illustrated in Fig. 15, and the filter size associated with the input noise reduction intensity β, 211 is acquired from the selected data, and the acquired filter size 213 is output to the average filter application unit 222.
The average filter application unit 222 applies an average filter having a size determined by the filter size 213 input from the LUT 221 to the fluorescence image 20 and executes the noise reduction processing on the fluorescence image.
A noise reduced fluorescence image 212 is generated according to this average filter application processing.
Note that, regarding the image feature amount, a configuration in which a single feature amount is acquired in the entire image including the visible light image 10 and the fluorescence image 20 or a configuration in which a filter having one size is applied to one image may be used. Furthermore, a configuration may be used in which the feature amount is individually acquired in image region unit and the filters having different sizes for the respective image regions are applied.
Note that, as in the embodiment described above, the filter applied when the noise reduced fluorescence image 212 is generated from the fluorescence image 20 is not limited to the average filter, and various filters such as a median filter, and a bilateral filter can be applied.
Furthermore, for example, a configuration can be used in which a joint bilateral filter using edge information is applied.
Next, a sequence of processing executed by the image processing apparatus 200 illustrated in Fig. 13 according to the present embodiment will be described with reference to the flowchart in Fig. 17.
Hereinafter, the flow illustrated in Fig. 17 is substantially similar to the flow in Fig. 12 described above.
Processing in steps S201, S202, and S204 in the flow illustrated in Fig. 17 is same as the processing in steps S201, S202, and S204 in the flow in Fig. 12.
In the flow in Fig. 17, only processing in step S203b is different from the processing in step S203 in the flow illustrated in Fig. 12.
The processing in step S203b in the flow in Fig. 17 will be described.

(Step S203b)

In step S203, the image processing apparatus applies the noise reduction intensity β calculated in step S202, executes the noise reduction processing on the fluorescence image, and generates the noise reduced fluorescence image.
This processing is executed by the fluorescence image noise reduction unit 205 of the image processing apparatus 200 illustrated in Fig. 13.

As described above with reference to Figs. 14 to 16, the fluorescence image noise reduction unit 205 includes the class classification processing unit 223, the look-up table (LUT) 221, and the average filter application unit 222.

The look-up table (LUT) 221 stores the noise reduction intensity $\beta$, 211 and an optimal average filter size associated with the class classification information 214.

The class classification processing unit 223 receives the visible light image 10 and the fluorescence image 20 and acquires the feature amounts of the two images. Next, the class classification information 214 is acquired on the basis of the feature amounts. The image features and the class classification information are associated with each other by applying the learning data to which the sample image imaged in advance is applied, and the class classification information 214 associated with the image feature amounts of the received visible light image 10 and fluorescence image 20 is selected and output by using the associated data.

Thereafter, according to the class classification information 214, a piece of data, in other words, one of the plurality of graphs illustrated in Fig. 15 is selected from among the LUTs 221 illustrated in Fig. 15, and the filter size associated with the input noise reduction intensity $\beta$, 211 is acquired from the selected data, and the acquired filter size 213 is output to the average filter application unit 222.

The average filter application unit 222 applies an average filter having a size determined by the filter size 213 input from the LUT 221 to the fluorescence image 20 and executes the noise reduction processing on the fluorescence image.

A noise reduced fluorescence image 212 is generated according to this average filter application processing.

(Step S204)

Next, in step S204, the image processing apparatus superimposes the visible light image with the noise reduced fluorescence image according to the superimposing rate $\alpha$ and generates the superimposed image.

In the present example, the noise signal level of the superimposed image changes according to a change in the superimposing rate $\alpha$. However, even when the superimposing rate $\alpha$ changes, the noise signal level (intensity) of the visible light image and the noise signal level (intensity) of the noise reduced fluorescence image forming the superimposed image are controlled to be set so that the noise signal levels substantially coincide with each other.

In other words, setting is controlled so that the noise level of the noise reduced fluorescence image constantly coincides with the noise level of the visible light image, and the superimposed image is displayed. Therefore, even when the superimposing rate of the superimposed image is changed and a ratio of the fluorescence image is changed, the increase or decrease of the noise is suppressed, and an image which can be easily observed can be displayed.

[5. Configuration and processing of third embodiment of image processing apparatus according to present disclosure]

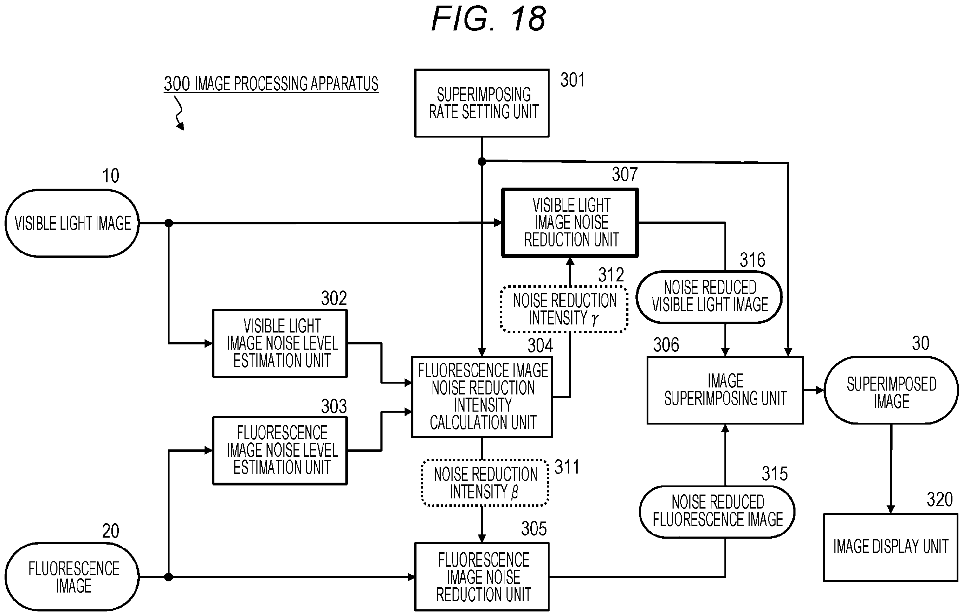

Next, a configuration and processing of a third embodiment of the image processing apparatus according to the present disclosure will be described with reference to Fig. 18 and subsequent drawings.

Fig. 18 is a block diagram illustrating an exemplary configuration of an image processing apparatus 300 according to the third embodiment of the present disclosure.

Similarly to the image processing apparatus 100 illustrated in Fig. 2 described as the first embodiment above, the image processing apparatus 300 according to the third embodiment illustrated in Fig. 18 receives a visible light image 10 and a fluorescence image 20 and generates a superimposed image 30 obtained by superimposing the two images and outputs the superimposed image 30. The superimposed image 30 is, for example, output to and displayed by an image display device 320 having an image display unit (display).

Similarly to the image processing apparatus 100 illustrated in Fig. 2 described as the first embodiment above, the image processing apparatus 300 illustrated in Fig. 18 has a configuration for controlling a noise level of the superimposed image 30 obtained by superimposing the visible light image 10 and the fluorescence image 20. Specifically, for example, a configuration is included which can lower the noise level of the fluorescence image 20 to the noise level of the visible light image 10 which is a superimposing target and superimposes the fluorescence image 20 and the visible light image 10.

As illustrated in Fig. 18, the image processing apparatus 300 includes a superimposing rate setting unit 301, a visible light image noise level estimation unit 302, a fluorescence image noise level estimation unit 303, a fluorescence image noise reduction intensity calculation unit 304, a fluorescence image noise reduction unit 305, and an image superimposing unit 306.

These components have the configurations similar to those of the components of the image processing apparatus 100 illustrated in Fig. 2 described as the first embodiment above.

A difference between the image processing apparatus 300 illustrated in Fig. 18 and the image processing apparatus 100 illustrated in Fig. 2 is that a visible light image noise reduction unit 307 is added.

In a case where a noise level of the input visible light image 10 is larger than a predetermined threshold (th), the visible light image noise reduction unit 307 executes processing for reducing the noise level of the visible light image 10, generates a noise reduced visible light image 316, and outputs the generated noise reduced visible light image 316 to the image superimposing unit 306.

In this case, the image superimposing unit 306 executes superimposition processing on two images including the noise reduced visible light image 316 generated by the visible light image noise reduction unit 307 and a noise reduced fluorescence image 315 generated by the fluorescence image noise reduction unit 305 and generates the superimposed image 30.

Note that a noise reduction intensity $\gamma$ 312 to be applied to the noise level reduction processing on the visible light image 10 by the visible light image noise reduction unit 307 is a fixed value which has been defined in advance.

As described above, in a case where the noise level of the input visible light image 10 is larger than the predetermined threshold, the visible light image noise reduction unit 307 executes processing for reducing the noise level of the visible light image 10.

The fluorescence image noise reduction intensity calculation unit 304 determines whether or not the noise level of the input visible light image 10 is larger than the predetermined threshold.

The fluorescence image noise reduction intensity calculation unit 304 determines whether or not the noise level of the input visible light image 10 is larger than the predetermined threshold. In a case where the fluorescence image noise reduction intensity calculation unit 304 determines that the noise level is larger than the threshold, the fluorescence image noise reduction intensity calculation unit 304 outputs the predetermined noise reduction intensity $\gamma$ 312 to the visible light image noise reduction unit 307.

The visible light image noise reduction unit 307 applies this noise reduction intensity $\gamma$ 312 and executes the noise level reduction processing on the visible light image 10.

The visible light image noise reduction unit 307 applies the noise level reduction intensity $\gamma$ to the input visible light image 10, in other words,

$$y_V = x_V + n_V$$

the visible light image 10 configured of the pixel value $y_V$ and generates the noise reduced visible light image 316 having a pixel value $y_{Vnr}$ from which the noise has been reduced according to the following formula.

$$y_{Vnr} = x_V + (1/\gamma) n_V$$

Note that, in the above formula, the signal $x_V$ is an original signal, in other words, an image signal which reflects a subject which does not include a noise component, and the signal $n_V$ is a signal including only a noise component different from the image signal which reflects the subject.

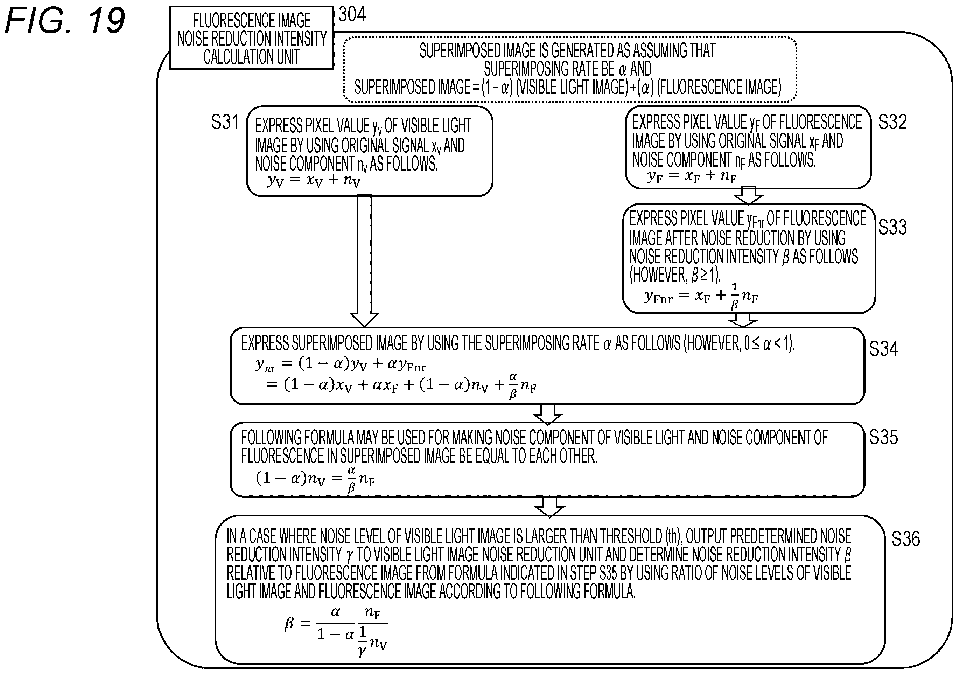

Next, processing executed by the fluorescence image noise reduction intensity calculation unit 304 will be described in detail with reference to Fig. 19.

Similarly to the description with reference to Fig. 5 in the first embodiment above, the fluorescence image noise reduction intensity calculation unit 304 calculates the noise level reduction intensity $\beta$, 311 which is a noise level reduction intensity of the fluorescence image 20 according to the noise levels of the visible light image 10 and the fluorescence image 20 respectively estimated by the visible light image noise level estimation unit 302 and the fluorescence image noise level estimation unit 303.

For example, even when the superimposing rates of the visible light image and the fluorescence image change, the fluorescence image noise reduction intensity calculation unit 304 calculates the noise level reduction intensity $\beta$, 311 according to the superimposing rate so that the noise levels of the visible light image and the fluorescence image included in the superimposed image substantially coincide with each other.

In the present embodiment, as described above, in a case where the noise level of the input visible light image 10 is larger than the predetermined threshold (th), the processing for reducing the noise level of the visible light image 10 is executed, and the noise reduced visible light image 316 is generated and output to the image superimposing unit 306.

In this case, the image superimposing unit 306 executes superimposition processing on two images including the noise reduced visible light image 316 generated by the visible light image noise reduction unit 307 and a noise reduced fluorescence image 315 generated by the fluorescence image noise reduction unit 305 and generates the superimposed image 30.

The image superimposing unit 306 superimposes the two images according to the superimposing rate $\alpha$ and generates the superimposed image 30. The pixel value $y_{nr}$ of the superimposed image 30 is expressed by the following formula (Formula 8).

$$y_{nr} = (1 - \alpha)y_{Vnr} + \alpha y_{Fnr}$$
$$= (1 - \alpha)x_V + \alpha x_F + (1 - \alpha)n_V + (\alpha/\beta)n_F \ldots (\text{Formula } 8)$$

In this way, in a case where the visible light image to be superimposed with the fluorescence image is the noise reduced visible light image 316, it is necessary to adjust the noise level of the noise reduced fluorescence image 315 to match the noise level of the noise reduced visible light image 316.

Fig. 19 is a processing sequence of this case, in other words, a processing sequence in a case where it is determined that the noise level of the input visible light image 10 is higher than the threshold and the processing for superimposing the noise reduced fluorescence image 315 with the noise reduced visible light image 316 is executed.

In other words, the sequence of the processing for adjusting the noise level of the noise reduced fluorescence image 315 to match the noise level of the noise reduced visible light image 316 is illustrated.

Hereinafter, processing in steps S31 to S36 executed by the fluorescence image noise reduction intensity calculation unit 304 will be sequentially described with reference to Fig. 19.

(Step S31)

First, in step S31, the fluorescence image noise reduction intensity calculation unit 304 expresses a pixel value $y_V$ of the visible light image by using an original signal $x_V$ and a noise component signal $n_V$ as the following formula.

$$y_V = x_V + n_V$$

Note that the original signal $x_V$ is an image signal which reflects a subject which does not include a noise component, and the noise component signal $n_V$ is a signal including only a noise component different from the image signal which reflects the subject.

(Step S32)

Next, in step S32, the fluorescence image noise reduction intensity calculation unit 304 expresses a pixel value $y_F$ of fluorescence image by using an original signal $x_F$ and a noise component $n_F$ as the following formula.

$$y_F = x_F + n_F$$

(Step S33)

Next, in step S33, the fluorescence image noise reduction intensity calculation unit 304 expresses a pixel value $y_{Fnr}$ of the fluorescence image from which the noise has been reduced by using the original signal $x_F$, the noise reduction intensity $\beta$, and the noise component $n_F$ as the following formula.

$$y_{Fnr} = x_F + (1/\beta)n_F$$

Note that, as indicated in the above formula, by the noise reduction processing to which the noise reduction intensity $\beta$ is applied, the noise component included in the pixel value of the fluorescence image is decreased to $(1/\beta)n_F$.

(Step S34)

Next, in step S34, the fluorescence image noise reduction intensity calculation unit 304 expresses a superimposed image $y_{nr}$ of the visible light image $y_V$ and the noise reduced fluorescence image $y_{Fnr}$ by using the superimposing rate $\alpha$ as follows.

$$y_{nr} = (1 - \alpha)y_V + \alpha y_{Fnr}$$
$$= (1 - \alpha)x_V + \alpha x_F + (1 - \alpha)n_V + (\alpha/\beta)n_F$$

(Step S35)

Next, in step S35, the fluorescence image noise reduction intensity calculation unit 304 sets a formula which makes the noise component $n_V$ of the visible light image and the noise component $(\alpha/\beta)n_F$ of the noise reduced fluorescence image forming the superimposed image be equal to each other, in other words, the following formula (Formula 4).

$$(1 - \alpha)n_V = (\alpha/\beta)n_F \ldots (\text{Formula 4})$$

(Formula 4) described above sets (a) a noise signal level of the visible light image $(1 - \alpha)n_V$ and (b) a noise signal level of the noise reduced fluorescence image $(\alpha/\beta)n_F$ included in the superimposed image to coincide with each other.

(Step S36)

Next, when confirming in step S36 that the noise level of the input visible light image 10 is larger than the threshold (th), the fluorescence image noise reduction intensity calculation unit 304 outputs a noise reduction intensity $\gamma$ for a visible light image which has been defined in advance to the visible light image noise reduction unit 307.

Moreover, by using (Formula 4) above, the noise reduction intensity $\beta$ to be applied to the noise reduction processing executed on the fluorescence image is calculated according to the following (Formula 9).

$$\beta = (\alpha/(1 - \alpha)) (n_F/((1/\gamma) n_V)) \ldots (\text{Formula 9})$$

(Formula 9) above corresponds to a formula in which
the noise level $n_V$ of the visible light image in the (Formula 5), in other words,

$$\beta = (\alpha/(1 - \alpha))(n_F/n_V) \ldots (\text{Formula 5})$$

a noise reduction intensity $\beta$ calculation formula which has been applied in step S26 described with reference to Fig. 5 in the first embodiment above,
into a noise level after the noise reduction processing, in other words, $(1/\gamma)n_V$.

In this way, the fluorescence image noise reduction intensity calculation unit 304 calculates the noise reduction intensity $\beta$ applied by the fluorescence image noise reduction unit 305 which executes the noise reduction processing on the fluorescence image 20.

As understood from (Formula 9) above, the noise reduction intensity $\beta$ is set to increase as the superimposing rate $\alpha$ of the fluorescence image increases and decrease as the superimposing rate $\alpha$ of the fluorescence image decreases.

The noise signal levels change according to a change in the superimposing rate $\alpha$. However, even when the superimposing rate $\alpha$ changes, the noise signal level (intensity) of the noise reduced visible light image and the noise signal level (intensity) of the noise reduced fluorescence image forming the superimposed image are controlled to be set so that the noise signal levels substantially coincide with each other.

The image superimposing unit 106 superimposes the two images according to the superimposing rate $\alpha$ and generates the superimposed image 30. The pixel value $y_{nr}$ of the superimposed image 30 is expressed by the formula (Formula 8) described above.

$$y_{nr} = (1 - \alpha) y_{Vnr} + \alpha y_{Fnr}$$
$$= (1 - \alpha) x_V + \alpha x_F + (1 - \alpha) n_V + (\alpha/\beta) n_F \ldots (\text{Formula 8})$$

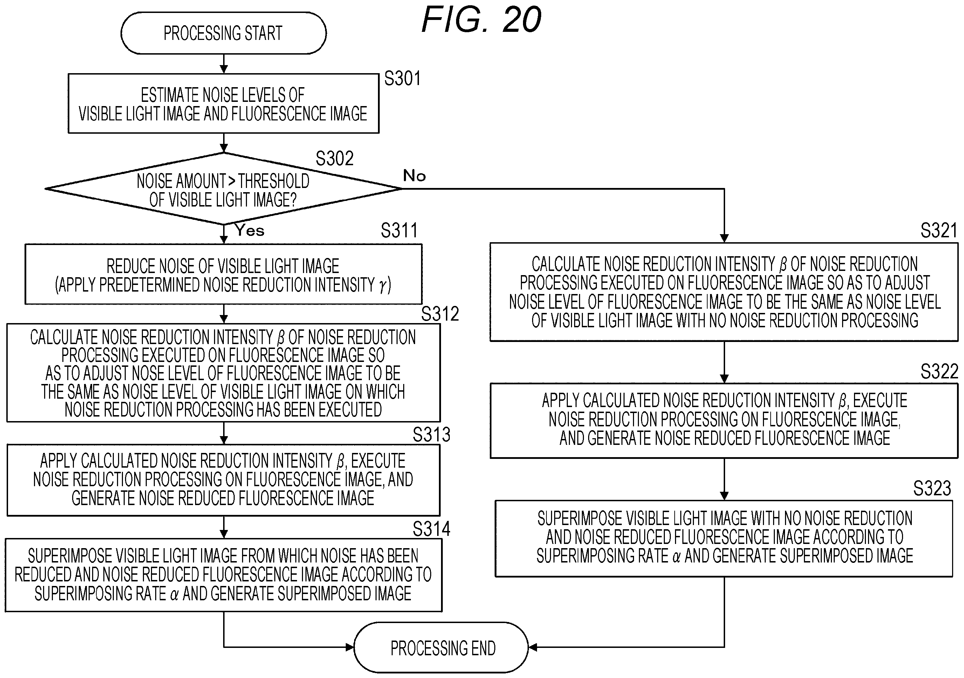

Next, a sequence of processing executed by the image processing apparatus 300 illustrated in Fig. 18 according to the third embodiment will be described with reference to the flowchart illustrated in Fig. 20.

Processing in each step of the flow illustrated in Fig. 20 will be sequentially described below.

(Step S301)

Processing in step S301 is similar to the processing in step S101 in the flowchart illustrated in Fig. 8 which is the processing according to the first embodiment described above.

First, the image processing apparatus estimates noise levels of the visible light image and the fluorescence image in step S301.

This processing is executed by the visible light image noise level estimation unit 302 and the fluorescence image noise level estimation unit 303 of the image processing apparatus 300 illustrated in Fig. 18.

The visible light image noise level estimation unit 302 and the fluorescence image noise level estimation unit 303 respectively estimate the noise levels included in the visible light image 10 and the fluorescence image 20 according to the sequence described with reference to Fig. 4 above.

(Step S302)

Next, in step S302, the image processing apparatus determines whether or not the noise level of the visible light image 10 is larger than the predetermined threshold (th) .

This processing is executed by the fluorescence image noise reduction intensity calculation unit 304 of the image processing apparatus 300 illustrated in Fig. 18.

In a case where the noise level of the visible light image 10 is larger than the predetermined threshold (th), the procedure proceeds to step S311.

On the other hand, in a case where the noise level of the visible light image 10 is not larger than the predetermined threshold (th), the procedure proceeds to step S321.

(Step S311)

In a case where the noise level of the visible light image 10 is larger than the predetermined threshold (th) in step S302, the procedure proceeds to step S311.

The image processing apparatus executes noise reduction processing on the visible light image in step S311.

This processing is executed by the visible light image noise reduction unit 307 of the image processing apparatus 300 illustrated in Fig. 18.

The visible light image noise reduction unit 307 applies the predetermined noise reduction intensity $\gamma$, executes the noise reduction processing on the visible light image, and generates a noise reduced visible light image.

(Step S312)

Next, in step S312, the image processing apparatus calculates the noise reduction intensity $\beta$ of the noise reduction processing executed on the fluorescence image so as to adjust the noise level of the fluorescence image to match the noise level of the noise reduced visible light image generated in step S311.

This processing is executed by the fluorescence image noise reduction intensity calculation unit 304 of the image processing apparatus 300 illustrated in Fig. 18.

This processing is the processing described with reference to Fig. 19 above.

As described with reference to Fig. 19, the fluorescence image noise reduction intensity calculation unit 304 executes the processing in steps S31 to S36 illustrated in Fig. 19 and calculates the noise level reduction intensity $\beta$ according to the superimposing rate so that the noise levels of the noise reduced visible light image and the noise reduced fluorescence image included in the superimposed image are substantially the same even when the superimposing rate $\alpha$ of the noise reduced visible light image and the noise reduced fluorescence image changes.

(Step S313)

Next, in step S313, the image processing apparatus applies the calculated noise reduction intensity $\beta$, executes the noise reduction processing on the fluorescence image, and generates the noise reduced fluorescence image.

This processing is executed by the fluorescence image noise reduction unit 305 of the image processing apparatus 300 illustrated in Fig. 18.

This processing is similar to the processing described with reference to Fig. 6 in the first embodiment.

Alternatively, the processing may be executed as the processing to which the look-up table (LUT) 221 described with reference to Fig. 10 and the like in the second embodiment is applied.

The fluorescence image noise reduction unit 305 of the image processing apparatus 300 illustrated in Fig. 18 executes any one of the processing and generates a noise reduced fluorescence image 312.

(Step S314)

Next, in step S314, the image processing apparatus superimposes the noise reduced visible light image with the noise reduced fluorescence image according to the superimposing rate $\alpha$ and generates the superimposed image.

This processing is executed by the image superimposing unit 306 of the image processing apparatus 300 illustrated in Fig. 18.

The generated superimposed image is displayed on the image display unit 220.

The image superimposing unit 106 superimposes the noise reduced visible light image on which the noise reduction processing is executed by applying the noise reduction intensity $\gamma$ with the noise reduced fluorescence image on which the noise reduction processing is executed by applying he noise reduction intensity $\gamma$ according to the superimposing rate $\alpha$ and generates the superimposed image 30. A pixel value $y_{nr}$ of the superimposed image 30 is expressed by the formula (Formula 8) described above.

(Step S321)

On the other hand, in a case where it is determined in step S302 that the noise level of the visible light image 10 is not larger than the predetermined threshold (th), the procedure proceeds to step S321.

The processing in steps S321 to S323 is similar to the processing in steps S102 to S104 described with reference to the flow in Fig. 8 in the first embodiment.

In step S321, the image processing apparatus calculates the noise reduction intensity $\beta$ of the noise reduction processing executed on the fluorescence image so as to adjust the noise level of the fluorescence image to match the noise level of the visible light image with no noise reduction.

This processing is executed by the fluorescence image noise reduction intensity calculation unit 304 of the image processing apparatus 300 illustrated in Fig. 18.

The processing in this case the processing in steps S21 to S26 described with reference to Fig. 5 in the first embodiment above.

Even when the superimposing rate $\alpha$ of the visible light image with no noise reduction and the noise reduced fluorescence image changes, the noise level reduction intensity $\beta$ according to the superimposing rate is calculated so that the noise levels of the visible light image with no noise reduction and the noise reduced fluorescence image included in the superimposed image are substantially the same.

(Step S322)

Next, in step S322, the image processing apparatus applies the calculated noise reduction intensity $\beta$, executes the noise reduction processing on the fluorescence image, and generates the noise reduced fluorescence image.

This processing is executed by the fluorescence image noise reduction unit 305 of the image processing apparatus 300 illustrated in Fig. 18.

This processing is similar to the processing described with reference to Fig. 6 in the first embodiment.

Alternatively, the processing may be executed as the processing to which the look-up table (LUT) 221 described with reference to Fig. 10 and the like in the second embodiment is applied.

The fluorescence image noise reduction unit 305 of the image processing apparatus 300 illustrated in Fig. 18 executes any one of the processing and generates a noise reduced fluorescence image 312.

(Step S323)

Next, in step S323, the image processing apparatus superimposes the visible light image with no noise reduction with the noise reduced fluorescence image according to the superimposing rate $\alpha$ and generates the superimposed image.

This processing is executed by the image superimposing unit 306 of the image processing apparatus 300 illustrated in Fig. 18.

This processing is similar to the processing described with reference to Fig. 7 in the first embodiment above. The image superimposing unit 306 superimposes the visible light image 10 with the noise reduced fluorescence image 312 generated by the fluorescence image noise reduction unit 305 and generates the superimposed image 30.

The generated superimposed image 30 is displayed on the image display unit 320.

As described above with reference to Fig. 7, the image superimposing unit 306 superimposes the visible light image 10 with the noise reduced fluorescence image 312 generated by the fluorescence image noise reduction unit 305 according to the superimposing rate $\alpha$ and generates the superimposed image 30. A pixel value $y_{nr}$ of the superimposed image 30 is indicated by the formula (Formula 7) described above.

In the present embodiment, in a case where the noise level of the visible light image is larger than the predetermined threshold, the superimposed image of the noise reduced visible light image and the noise reduced fluorescence image is generated, and in a case where the noise level of the visible light image is not larger than the predetermined threshold, the superimposed image of the visible light image with no noise reduction and the noise reduced fluorescence image is generated.

However, in any case, the noise signal level of the superimposed image changes according to the change in the superimposing rate α. However, even when the superimposing rate α changes, the noise signal level (intensity) of the visible light image or the noise reduced visible light image included in the superimposed image and the noise signal level (intensity) of the noise reduced fluorescence image are controlled to be set so that the noise signal levels substantially coincide with each other.

In other words, the noise level of the noise reduced fluorescence image is constantly controlled to be set to coincide with the noise level of the visible light image to be superimposed or the noise reduced visible light image and is displayed. Therefore, even when the superimposing rate is changed and the ratio of the fluorescence image is changed, increase or decrease in the noise is suppressed, and it is possible to display an image which can be easily observed.

[6. Exemplary hardware configuration of image processing apparatus]

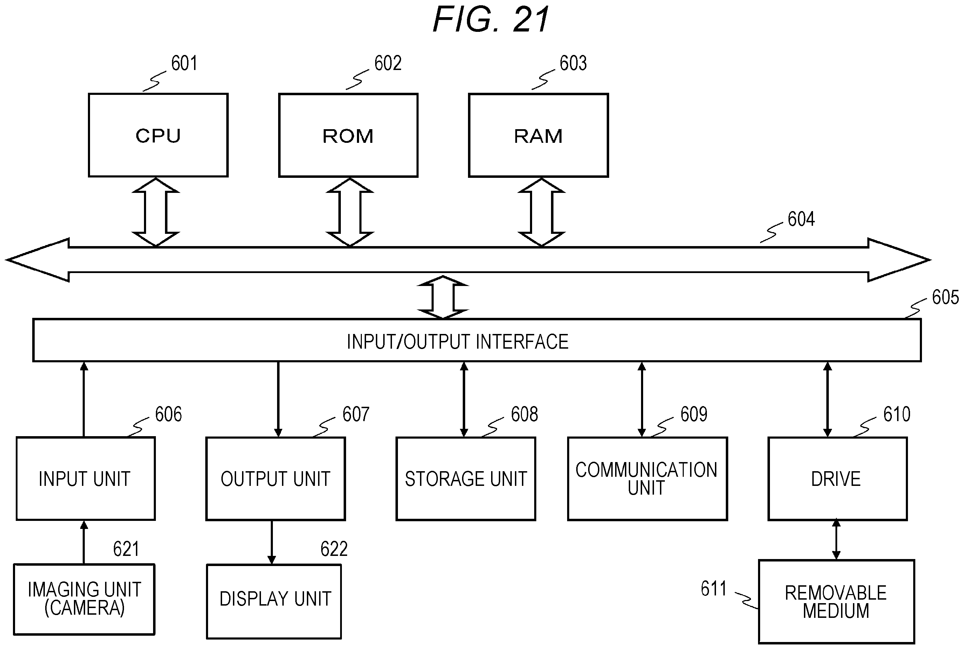

Next, an exemplary hardware configuration of the image processing apparatus will be described with reference to Fig. 21.

Fig. 21 is a diagram illustrating an exemplary hardware configuration of the image processing apparatus for executing processing according to the present disclosure.

A Central Processing Unit (CPU) 601 functions as a control unit and a data processing unit which execute various processing according to a program stored in a Read Only Memory (ROM) 602 or a storage unit 608. For example, processing according to the sequence described in the above embodiment is executed. A Random Access Memory (RAM) 603 stores the program executed by the CPU 601, data, and the like. The CPU 601, the ROM 602, and the RAM 603 are connected to each other by a bus 604.

The CPU 601 is connected to an input/output interface 605 via the bus 604. The input/output interface 605 is connected to an input unit 606 which inputs an imaged image of an imaging unit 621 and includes various switches, a keyboard, a mouse, a microphone, and the like to which a user can make an input and an output unit 607 which outputs data to a display unit 622, a speaker, and the like. The CPU 601 executes various processing in response to an instruction input from the input unit 606 and outputs the processing result to, for example, the output unit 607.

The storage unit 608 connected to the input/output interface 605 includes, for example, a hard disk and the like and stores the program executed by the CPU 601 and various data. A communication unit 609 functions as a transceiver for data communication via a network such as Wi-Fi communication, Bluetooth (registered trademark) communication, other Internet, and a local area network and communicates with external devices.

A drive 610 connected to the input/output interface 605 drives a removable medium 611 such as a magnetic disk, an optical disk, a magneto-optical disk, or a semiconductor memory such as a memory card and records or reads data.

[7. Summary of configuration of present disclosure]

The embodiments of the present disclosure have been described in detail with reference to the specific embodiments above. However, it is obvious that those skilled in the art can make modifications and substitutions of the embodiments without departing from the gist of the present disclosure. In other words, the present invention has been disclosed in a form of exemplification and is not restrictively interpreted. Claims should be considered in order to determine the gist of the present disclosure.

Note that the technology disclosed in the present specification can have the following configuration.

(1) An image processing apparatus including:

a fluorescence image noise reduction unit configured to execute noise reduction processing on a fluorescence image and generate a noise reduced fluorescence image; and
an image superimposing unit configured to generate a superimposed image of a visible light image with the noise reduced fluorescence image, in which
the noise reduction processing includes processing for adjusting a noise level of the fluorescence image to be closer to a noise level of the visible image forming the superimposed image.

(2) The image processing apparatus according to (1), further including:

a fluorescence image noise reduction intensity calculation unit configured to calculate a noise reduction intensity to be applied to the noise reduction processing, in which

the fluorescence image noise reduction intensity calculation unit calculates a noise reduction intensity having a value which makes a noise level of the noise reduced fluorescence image substantially coincide with the noise level of the visible light image forming the superimposed image.

(3) The image processing apparatus according to (2), further including:

a visible light image noise level estimation unit configured to estimate the noise level of the visible light image; and a fluorescence image noise level estimation unit configured to estimate the noise level of the fluorescence image, in which

the fluorescence image noise reduction intensity calculation unit calculates the noise reduction intensity by applying the noise level of the visible light image and the noise level of the fluorescence image.

(4) The image processing apparatus according to (2) or (3), further including:

a superimposing rate setting unit configured to set a superimposing rate of each image which is applied to superimposition processing of a visible light image and a noise reduced fluorescence image executed by the image superimposing unit, in which

the fluorescence image noise reduction intensity calculation unit calculates a noise reduction intensity having a value which makes a noise level of the noise reduced fluorescence image substantially coincide with the noise level of the visible light image forming the superimposed image even in a case where the superimposing rate is changed.

(5) The image processing apparatus according to (4), in which
the fluorescence image noise reduction intensity calculation unit calculates a noise reduction intensity that increases as the superimposing rate of the noise reduced fluorescence image increases.
(6) The image processing apparatus according to (4), further including:

a visible light image noise level estimation unit configured to estimate the noise level of the visible light image; and a fluorescence image noise level estimation unit configured to estimate the noise level of the fluorescence image, in which

the fluorescence image noise reduction intensity calculation unit calculates the noise reduction intensity by applying the noise level of the visible light image, the noise level of the fluorescence image, and the superimposing rate.

(7) The image processing apparatus according to any one of (1) to (6), in which
the fluorescence image noise reduction unit includes a look-up table including corresponding data of a noise reduction intensity and a filter size, and
acquires a filter size associated with the noise reduction intensity calculated by a fluorescence image noise reduction intensity calculation unit from the look-up table, applies a filter having the acquired filter size to a fluorescence image, and generates a noise reduced fluorescence image.
(8) The image processing apparatus according to (7), in which
the filter includes any one of an average filter, a median filter, or a bilateral filter.
(9) The image processing apparatus according to (7) or (8), in which
the fluorescence image noise reduction unit includes
a class classification processing unit that outputs class classification information based on an image feature amount to which prior learning data is applied, and
the class classification processing unit
outputs class classification information based on feature amounts of a visible light image and a fluorescence image for superimposition processing,
applies a look-up table selected according to the class classification information, acquires the filter size associated with the noise reduction intensity, applies the filter having the acquired filter size to the fluorescence image, and generates a noise reduced fluorescence image.
(10) The image processing apparatus according to any one of (1) to (9), further including:

a visible light image noise reduction unit, in which
the visible light image noise reduction unit executes noise reduction processing on the visible light image and generates a noise reduced visible image in a case where the visible light image has a noise level larger than a predetermined threshold, and

the image superimposing unit generates a superimposed image of the noise reduced visible light image and the noise reduced fluorescence image.

(11) The image processing apparatus according to (10), in which

the fluorescence image noise reduction intensity calculation unit calculates a noise reduction intensity having a value which makes a noise level of the noise reduced fluorescence image substantially coincide with a noise level of the noise reduced visible light image forming the superimposed image.

(12) The image processing apparatus according to any one of (1) to (11), further including:

an imaging unit configured to execute processing for imaging the visible light image and the fluorescence image.

(13) An image processing method executed by an image processing apparatus, the method including:

a fluorescence image noise reduction step for executing noise reduction processing on a fluorescence image and generating a noise reduced fluorescence image by a fluorescence image noise reduction unit; and

an image superimposing step for generating a superimposed image of a visible light image and the noise reduced fluorescence image by an image superimposing unit, in which

the noise reduction processing executed in the fluorescence image noise reduction step includes processing for adjusting a noise level of the fluorescence image to be closer to a noise level of the visible image forming the superimposed image.

(14) A program for causing an image processing apparatus to execute image processing including:

a fluorescence image noise reduction step for causing a fluorescence image noise reduction unit to execute noise reduction processing on a fluorescence image and generate a noise reduced fluorescence image; and

an image superimposing step for causing an image superimposing unit to generate a superimposed image of a visible light image and the noise reduced fluorescence image, in which

the noise reduction processing executed in the fluorescence image noise reduction step is executed as processing adjusting a noise level of the fluorescence image to be closer to a noise level of the visible image forming the superimposed image.

Furthermore, the series of processing described in the specification can be executed by hardware, software, or a composite configuration of the hardware and the software. In a case where the processing is executed by the software, it is possible that a program in which a processing sequence has been recorded is installed in a memory, which is built in a dedicated hardware, in a computer and executed or the program is installed in a general computer which can execute various processing and executed. For example, the program can be recorded in a recording medium in advance. In addition to installing the program from the recording medium to a computer, it is possible that the program is received via a network such as a Local Area Network (LAN) and the Internet and installed to a recording medium such as a built-in hard disk.

Note that various processing described in the present specification is not only executed in time series according to the description, and may be executed in parallel or individually according to a processing capability of an apparatus for executing the processing or as necessity. Furthermore, in the present specification, the system is a logical group configuration of a plurality of devices, and the devices of the configuration are not limited to be housed in the same casing.

INDUSTRIAL APPLICABILITY

As described above, according to one embodiment of the present disclosure, an apparatus and a method are realized for generating a superimposed image of a visible light image and a fluorescence image having a noise level which substantially coincides with a noise level of the visible light image.

Specifically, for example, a fluorescence image noise reduction unit which executes noise reduction processing on the fluorescence image and generates a noise reduced fluorescence image, an image superimposing unit which generates a superimposed image of the visible light image and the noise reduced fluorescence image according to the set superimposing rate, and a fluorescence image noise reduction intensity calculation unit which calculates a noise reduction intensity to be applied to the noise reduction processing on the fluorescence image by the fluorescence image noise reduction unit. Even in a case where the superimposing rate is changed, the fluorescence image noise reduction intensity calculation unit calculates a noise reduction intensity having a value which makes a noise level of the noise reduced fluorescence image substantially coincide with a noise level of the visible light image forming the superimposed image.

With these configuration and processing, an apparatus and a method are realized for generating a superimposed image of a visible light image and a fluorescence image having a noise level which substantially coincides with a noise level of the visible light image.

REFERENCE SIGNS LIST

1 Body tissue
2 Blood vessel
10 Visible light image
20 Fluorescence image
30 Superimposed image
100, 200, 300 Image processing apparatus
101, 201, 301 Superimposing rate setting unit
102, 202, 302 Visible light image noise level estimation unit
103, 203, 303 Fluorescence image noise level estimation unit
104, 204, 304 Fluorescence image noise reduction intensity calculation unit
105, 205, 405 Fluorescence image noise reduction unit
106, 206, 306 Image superimposing unit
221 Look-up table (LUT)
222 Average filter application unit
223 Class classification processing unit
307 Visible light image noise reduction unit
601 CPU
602 ROM
603 RAM
604 Bus
605 Input/output interface
606 Input unit
607 Output unit
608 Storage unit
609 Communication unit
610 Drive
611 Removable medium
621 Imaging unit
622 Display unit

**Claims**

1. An image processing apparatus comprising:

   a fluorescence image noise reduction unit configured to execute noise reduction processing on a fluorescence image and generate a noise reduced fluorescence image; and
   an image superimposing unit configured to generate a superimposed image of a visible light image with the noise reduced fluorescence image, wherein
   the noise reduction processing includes processing for adjusting a noise level of the fluorescence image to be closer to a noise level of the visible image forming the superimposed image.

2. The image processing apparatus according to claim 1, further comprising:

   a fluorescence image noise reduction intensity calculation unit configured to calculate a noise reduction intensity to be applied to the noise reduction processing, wherein
   the fluorescence image noise reduction intensity calculation unit calculates a noise reduction intensity having a value which makes a noise level of the noise reduced fluorescence image substantially coincide with the noise level of the visible light image forming the superimposed image.

**3.** The image processing apparatus according to claim 2, further comprising:

a visible light image noise level estimation unit configured to estimate the noise level of the visible light image; and
a fluorescence image noise level estimation unit configured to estimate the noise level of the fluorescence image, wherein
the fluorescence image noise reduction intensity calculation unit calculates the noise reduction intensity by applying the noise level of the visible light image and the noise level of the fluorescence image.

**4.** The image processing apparatus according to claim 2, further comprising:

a superimposing rate setting unit configured to set a superimposing rate of each image which is applied to superimposition processing of a visible light image and a noise reduced fluorescence image executed by the image superimposing unit, wherein
the fluorescence image noise reduction intensity calculation unit calculates a noise reduction intensity having a value which makes a noise level of the noise reduced fluorescence image substantially coincide with the noise level of the visible light image forming the superimposed image even in a case where the superimposing rate is changed.

**5.** The image processing apparatus according to claim 4, wherein
the fluorescence image noise reduction intensity calculation unit calculates a noise reduction intensity that increases as the superimposing rate of the noise reduced fluorescence image increases.

**6.** The image processing apparatus according to claim 4, further comprising:

a visible light image noise level estimation unit configured to estimate the noise level of the visible light image; and
a fluorescence image noise level estimation unit configured to estimate the noise level of the fluorescence image, wherein
the fluorescence image noise reduction intensity calculation unit calculates the noise reduction intensity by applying the noise level of the visible light image, the noise level of the fluorescence image, and the superimposing rate.

**7.** The image processing apparatus according to claim 1, wherein

the fluorescence image noise reduction unit includes a look-up table including corresponding data of a noise reduction intensity and a filter size, and
acquires a filter size associated with the noise reduction intensity calculated by a fluorescence image noise reduction intensity calculation unit from the look-up table, applies a filter having the acquired filter size to a fluorescence image, and generates a noise reduced fluorescence image.

**8.** The image processing apparatus according to claim 7, wherein
the filter includes any one of an average filter, a median filter, or a bilateral filter.

**9.** The image processing apparatus according to claim 7, wherein

the fluorescence image noise reduction unit includes
a class classification processing unit that outputs class classification information based on an image feature amount to which prior learning data is applied, and
the class classification processing unit
outputs class classification information based on feature amounts of a visible light image and a fluorescence image for superimposition processing,
applies a look-up table selected according to the class classification information, acquires the filter size associated with the noise reduction intensity, applies the filter having the acquired filter size to the fluorescence image, and generates a noise reduced fluorescence image.

**10.** The image processing apparatus according to claim 1, further comprising:

a visible light image noise reduction unit, wherein
the visible light image noise reduction unit executes noise reduction processing on the visible light image and generates a noise reduced visible image in a case where the visible light image has a noise level larger than a predetermined threshold, and
the image superimposing unit generates a superimposed image of the noise reduced visible light image and the noise reduced fluorescence image.

11. The image processing apparatus according to claim 10, wherein
the fluorescence image noise reduction intensity calculation unit calculates a noise reduction intensity having a value which makes a noise level of the noise reduced fluorescence image substantially coincide with a noise level of the noise reduced visible light image forming the superimposed image.

12. The image processing apparatus according to claim 1, further comprising:
an imaging unit configured to execute processing for imaging the visible light image and the fluorescence image.

13. An image processing method executed by an image processing apparatus, the method comprising:

a fluorescence image noise reduction step for executing noise reduction processing on a fluorescence image and generating a noise reduced fluorescence image by a fluorescence image noise reduction unit; and
an image superimposing step for generating a superimposed image of a visible light image and the noise reduced fluorescence image by an image superimposing unit, wherein
the noise reduction processing executed in the fluorescence image noise reduction step includes processing for adjusting a noise level of the fluorescence image to be closer to a noise level of the visible image forming the superimposed image.

14. A program for causing an image processing apparatus to execute image processing including:

a fluorescence image noise reduction step for causing a fluorescence image noise reduction unit to execute noise reduction processing on a fluorescence image and generate a noise reduced fluorescence image; and
an image superimposing step for causing an image superimposing unit to generate a superimposed image of a visible light image and the noise reduced fluorescence image, wherein
the noise reduction processing executed in the fluorescence image noise reduction step is executed as processing adjusting a noise level of the fluorescence image to be closer to a noise level of the visible image forming the superimposed image.

# FIG. 1

(1) EXEMPLARY IMAGING CONFIGURATION 1 OF FLUORESCENCE IMAGE

EXCITATION LIGHT  FLUORESCENCE

SCATTERED LIGHT  SCATTERED LIGHT

1 BODY TISSUE

2 BLOOD VESSEL

(2) EXEMPLARY IMAGING CONFIGURATION 2 OF FLUORESCENCE IMAGE

EXCITATION LIGHT  FLUORESCENCE

SCATTERED LIGHT  SCATTERED LIGHT

1 BODY TISSUE  2 BLOOD VESSEL

EP 3 590 404 A1

# FIG. 2

## FIG. 3

**SUPERIMPOSING RATE SETTING UNIT** 101

121 (ADJUSTED BY IMAGE OBSERVER)

SUPERIMPOSING RATE $\alpha$

0%
VISIBLE IMAGE (COLOR IMAGE)

50%
SUPERIMPOSED IMAGE OF VISIBLE IMAGE AND FLUORESCENCE IMAGE

100%
FLUORESCENCE IMAGE (BLACK-AND-WHITE TWO-VALUE IMAGE)

TO EASILY DISTINGUISH FLUORESCENCE IMAGE IN SUPERIMPOSED IMAGE FROM COLOR VISIBLE IMAGE, BLACK-AND-WHITE TWO-VALUE FLUORESCENCE IMAGE MAY BE COLORED (FOR EXAMPLE, GREEN) AND SUPERIMPOSED

SUPERIMPOSED IMAGE IS EXPRESSED BY USING SUPERIMPOSING RATE $\alpha$ AS FOLLOWS (HOWEVER, $0 \leq \alpha < 1$)

$$y_{nr} = (1 - \alpha)y_{V} + \alpha y_{Fnr}$$

SUPERIMPOSING RATE MAY BE SET FOR EACH IMAGE REGION

TO ACCURATELY AND QUICKLY CONFIRM LESION PORTION FROM VISIBLE IMAGE AND FLUORESCENCE IMAGE, OBSERVER CAN OBSERVE IMAGE AS CHANGING SUPERIMPOSING RATE. SUPERIMPOSING RATE CAN BE ADJUSTED BY OBSERVER. ALTERNATIVELY, SUPERIMPOSING RATE CAN BE SET IN ADVANCE ACCORDING TO OBSERVING PORTION AND THE LIKE.

AT THE TIME OF SUPERIMPOSITION PROCESSING, IT IS PREFERABLE TO SUPERIMPOSE FLUORESCENCE IMAGE AS DATA WHICH CAN BE EASILY DISTINGUISH FROM VISIBLE IMAGE SIGNAL, FOR EXAMPLE, BY COLORING FLUORESCENCE IMAGE WITH GREEN AND THE LIKE.

# FIG. 4

| VISIBLE LIGHT IMAGE NOISE LEVEL ESTIMATION UNIT | 102 |

| FLUORESCENCE IMAGE NOISE LEVEL ESTIMATION UNIT | 103 |

**S11**

SELECT FLAT IMAGE REGION BLOCK FROM VISIBLE LIGHT IMAGE

$$(x_{vn}, y_{vn}) \quad n = 0,1,2,3...$$

**S12**

SELECT LOW LUMINANCE IMAGE REGION BLOCK FROM FLUORESCENCE IMAGE

$$(x_{fm}, y_{fm}) \quad m = 0,1,2,3...$$

**S13**

PAIR OF TWO BLOCKS POSITIONED CLOSEST TO EACH OTHER IN FLAT IMAGE REGION BLOCK IN VISIBLE LIGHT IMAGE AND LOW LUMINANCE IMAGE REGION BLOCK IN FLUORESCENCE IMAGE ARE ASSUMED AS NOISE MEASUREMENT BLOCKS

$$(x_{min}, y_{min}) = \min(\sum_{i=0}^{m}\sum_{k=0}^{n} |x_{vn} - x_{fm}| + |y_{vn} - y_{fm}|)$$

**S14**

IN A CASE WHERE THERE IS PLURALITY OF NOISE MEASUREMENT BLOCKS, BLOCK CLOSEST TO CENTER OF IMAGE IS FURTHER SELECTED AS NOISE MEASUREMENT REGION

$$(x_{min}, y_{min}) = \min(\sum_{i=0}^{m}\sum_{k=0}^{n} |x_{min}| + |y_{min}|)$$

**S15**

CALCULATE STANDARD DEVIATION OF PIXEL VALUE OF SELECTED BLOCK OF VISIBLE LIGHT IMAGE AND ESTIMATE NOISE LEVEL ON THE BASIS OF CALCULATED STANDARD DEVIATION

**S16**

CALCULATE STANDARD DEVIATION OF PIXEL VALUE OF SELECTED BLOCK OF FLUORESCENCE IMAGE AND ESTIMATE NOISE LEVEL ON THE BASIS OF CALCULATED STANDARD DEVIATION

ACCORDING TO ABOVE PROCESSING, STANDARD DEVIATION OF LUMINANCE IN SELECTED BLOCK IS CALCULATED, AND NOISE LEVEL IS ESTIMATED ON THE BASIS OF CALCULATED STANDARD DEVIATION (NOISE LEVEL INCREASES AS STANDARD DEVIATION INCREASES) NOTE THAT, FOR CALCULATION OF NOISE LEVEL, AVERAGE VALUE, VARIANCE, SUM OF ADJACENT DIFFERENCE ABSOLUTE VALUES, OR THE LIKE MAY BE USED IN ADDITION TO STANDARD DEVIATION.

## FIG. 5

| FLUORESCENCE IMAGE NOISE REDUCTION INTENSITY CALCULATION UNIT | 104 |

SUPERIMPOSED IMAGE IS GENERATED AS ASSUMING THAT SUPERIMPOSING RATE BE $\alpha$ AND
SUPERIMPOSED IMAGE = $(1 - \alpha)$ (VISIBLE LIGHT IMAGE) + $(\alpha)$ (FLUORESCENCE IMAGE)

S21 EXPRESS PIXEL VALUE $y_V$ OF VISIBLE LIGHT IMAGE BY USING ORIGINAL SIGNAL $x_V$ AND NOISE COMPONENT $n_V$ AS FOLLOWS.
$$y_V = x_V + n_V$$

S22 EXPRESS PIXEL VALUE $y_F$ OF FLUORESCENCE IMAGE BY USING ORIGINAL SIGNAL $x_F$ AND NOISE COMPONENT $n_F$ AS FOLLOWS.
$$y_F = x_F + n_F$$

S23 EXPRESS PIXEL VALUE $y_{Fnr}$ OF FLUORESCENCE IMAGE AFTER NOISE REDUCTION BY USING NOISE REDUCTION INTENSITY $\beta$ AS FOLLOWS (HOWEVER, $\beta \geq 1$).
$$y_{Fnr} = x_F + \frac{1}{\beta} n_F$$

S24 EXPRESS SUPERIMPOSED IMAGE BY USING THE SUPERIMPOSING RATE $\alpha$ AS FOLLOWS (HOWEVER, $0 \leq \alpha < 1$).
$$y_{nr} = (1 - \alpha)y_V + \alpha y_{Fnr}$$
$$= (1 - \alpha)x_V + \alpha x_F + (1 - \alpha)n_V + \frac{\alpha}{\beta} n_F \frac{1}{3} n_F$$

S25 FOLLOWING FORMULA MAY BE USED FOR MAKING NOISE COMPONENT OF VISIBLE LIGHT AND NOISE COMPONENT OF FLUORESCENCE IN SUPERIMPOSED IMAGE BE EQUAL TO EACH OTHER.
$$(1 - \alpha)n_V = \frac{\alpha}{\beta} n_F$$

S26 FROM FORMULA ILLUSTRATED IN STEP S25, NOISE REDUCTION INTENSITY $\beta$ RELATIVE TO FLUORESCENCE IMAGE IS DETERMINED ACCORDING TO FOLLOWING FORMULA BY USING RATIO BETWEEN NOISE LEVELS OF VISIBLE LIGHT IMAGE AND FLUORESCENCE IMAGE.
$$\beta = \frac{\alpha}{1 - \alpha} \frac{n_F}{n_V}$$

EP 3 590 404 A1

## FIG. 6

**104**

FLUORESCENCE IMAGE
NOISE REDUCTION INTENSITY
CALCULATION UNIT

**111**

NOISE REDUCTION INTENSITY
$\beta$

**105**

FLUORESCENCE
IMAGE NOISE
REDUCTION UNIT

**20**

FLUORESCENCE
IMAGE

$$y_{\mathrm{F}} = x_{\mathrm{F}} + n_{\mathrm{F}}$$

PIXEL VALUE $y_{\mathrm{Fnr}}$ OF FLUORESCENCE IMAGE AFTER
NOISE REDUCTION IS CORRECTED AS FOLLOWS BY
USING ORIGINAL SIGNAL $x_{\mathrm{F}}$ AND NOISE COMPONENT $n_{\mathrm{F}}$
IN FLUORESCENCE IMAGE ($y_{\mathrm{F}} = x_{\mathrm{F}} + n_{\mathrm{F}}$) AND NOISE
REDUCTION INTENSITY $\beta$ (HOWEVER, $\beta \geq 1$).

$$y_{\mathrm{Fnr}} = x_{\mathrm{F}} + \frac{1}{\beta} n_{\mathrm{F}}$$

**112**

NOISE REDUCED
FLUORESCENCE
IMAGE

$$y_{\mathrm{Fnr}} = x_{\mathrm{F}} + \frac{1}{\beta} n_{\mathrm{F}}$$

# FIG. 7

$$y_V = x_V + n_V$$

SUPERIMPOSING RATE SETTING UNIT  101

SUPERIMPOSING RATE $\alpha$

VISIBLE LIGHT IMAGE  10

IMAGE SUPERIMPOSING UNIT  106

$y_{nr}$

SUPERIMPOSED IMAGE  30

NOISE REDUCED FLUORESCENCE IMAGE  112

$$y_{nr} = (1 - \alpha)y_V + \alpha y_{Fnr}$$
$$= (1 - \alpha)x_V + \alpha x_F + (1 - \alpha)n_V + \frac{\alpha}{\beta}n_F$$

$$y_{Fnr} = x_F + \frac{1}{\beta}n_F$$

IMAGE DISPLAY UNIT  120

EP 3 590 404 A1

# FIG. 8

PROCESSING START

S101

ESTIMATE NOISE LEVELS OF
VISIBLE LIGHT IMAGE AND FLUORESCENCE IMAGE

S102

CALCULATE NOISE REDUCTION INTENSITY $\beta$ OF
NOISE REDUCTION PROCESSING EXECUTED ON
FLUORESCENCE IMAGE SO AS TO ADJUST
NOISE LEVEL OF FLUORESCENCE IMAGE TO BE
THE SAME AS NOISE LEVEL OF VISIBLE LIGHT IMAGE

S103

APPLY CALCULATED NOISE REDUCTION INTENSITY $\beta$,
EXECUTE NOISE REDUCTION PROCESSING ON
FLUORESCENCE IMAGE, AND GENERATE
NOISE REDUCED FLUORESCENCE IMAGE

S104

SUPERIMPOSE VISIBLE LIGHT IMAGE AND NOISE REDUCED
FLUORESCENCE IMAGE ACCORDING TO SUPERIMPOSING
RATE $\alpha$ AND GENERATE SUPERIMPOSED IMAGE

PROCESSING END

# FIG. 9

200 IMAGE PROCESSING APPARATUS

201
SUPERIMPOSING RATE SETTING UNIT

10
VISIBLE LIGHT IMAGE

202
VISIBLE LIGHT IMAGE NOISE LEVEL ESTIMATION UNIT

203
FLUORESCENCE IMAGE NOISE LEVEL ESTIMATION UNIT

204
FLUORESCENCE IMAGE NOISE REDUCTION INTENSITY CALCULATION UNIT

211
NOISE REDUCTION INTENSITY $\beta$

206
IMAGE SUPERIMPOSING UNIT

30
SUPERIMPOSED IMAGE

212
NOISE REDUCED FLUORESCENCE IMAGE

220
IMAGE DISPLAY UNIT

20
FLUORESCENCE IMAGE

205
FLUORESCENCE IMAGE NOISE REDUCTION UNIT

221
LUT

EP 3 590 404 A1

# FIG. 10

EP 3 590 404 A1

# FIG. 11

FILTER SIZE

NOISE REDUCTION INTENSITY $\beta$

221

LUT

## FIG. 12

PROCESSING START

S201

ESTIMATE NOISE LEVELS OF
VISIBLE LIGHT IMAGE AND FLUORESCENCE IMAGE

S202

CALCULATE NOISE REDUCTION INTENSITY $\beta$ OF
NOISE REDUCTION PROCESSING EXECUTED ON
FLUORESCENCE IMAGE SO AS TO ADJUST
NOISE LEVEL OF FLUORESCENCE IMAGE TO BE
THE SAME AS NOISE LEVEL OF VISIBLE LIGHT IMAGE

S203

APPLY FILTER SELECTED ON THE BASIS OF CALCULATED
NOISE REDUCTION INTENSITY $\beta$, EXECUTE NOISE REDUCTION
PROCESSING ON FLUORESCENCE IMAGE, AND
GENERATE NOISE REDUCED FLUORESCENCE IMAGE

S204

SUPERIMPOSE VISIBLE LIGHT IMAGE AND NOISE REDUCED
FLUORESCENCE IMAGE ACCORDING TO SUPERIMPOSING
RATE $\alpha$ AND GENERATE SUPERIMPOSED IMAGE

PROCESSING END

EP 3 590 404 A1

# FIG. 13

200 IMAGE PROCESSING APPARATUS

201
SUPERIMPOSING
RATE SETTING UNIT

10
VISIBLE LIGHT IMAGE

202
VISIBLE LIGHT
IMAGE NOISE LEVEL
ESTIMATION UNIT

203
FLUORESCENCE
IMAGE NOISE LEVEL
ESTIMATION UNIT

204
FLUORESCENCE IMAGE
NOISE REDUCTION
INTENSITY
CALCULATION UNIT

211
NOISE REDUCTION
INTENSITY β

206
IMAGE
SUPERIMPOSING UNIT

212
NOISE REDUCED
FLUORESCENCE IMAGE

30
SUPERIMPOSED
IMAGE

220
IMAGE DISPLAY UNIT

20
FLUORESCENCE
IMAGE

205
FLUORESCENCE
IMAGE NOISE
REDUCTION UNIT

221
LUT

EP 3 590 404 A1

# FIG. 14

EP 3 590 404 A1

FIG. 15

EP 3 590 404 A1

## FIG. 16

| (a) IMAGE FEATURES | | (b) CLASS CLASSIFICATION INFORMATION | (c) APPLIED LUT |
|---|---|---|---|
| (1) FLAT PORTION | FLAT LEVEL (WEAK) | 101 (FLAT PORTION 01) | CLASS 101 CORRESPONDING NOISE INTENSITY — FILTER SIZE LUT |
| | FLAT LEVEL (MEDIUM) | 102 (FLAT PORTION 02) | CLASS 102 CORRESPONDING NOISE INTENSITY — FILTER SIZE LUT |
| | FLAT LEVEL (STRONG) | 103 (FLAT PORTION 03) | CLASS 103 CORRESPONDING NOISE INTENSITY — FILTER SIZE LUT |
| (2) TEXTURE PORTION | TEXTURE LEVEL (WEAK) | 201 (TEXTURE PORTION 01) | CLASS 201 CORRESPONDING NOISE INTENSITY — FILTER SIZE LUT |
| | TEXTURE LEVEL (MEDIUM) | 202 (TEXTURE PORTION 02) | CLASS 202 CORRESPONDING NOISE INTENSITY — FILTER SIZE LUT |
| | TEXTURE LEVEL (STRONG) | 203 (TEXTURE PORTION 03) | CLASS 203 CORRESPONDING NOISE INTENSITY — FILTER SIZE LUT |
| (3) EDGE PORTION | EDGE LEVEL (WEAK) | 301 (EDGE PORTION 01) | CLASS 301 CORRESPONDING NOISE INTENSITY — FILTER SIZE LUT |
| | EDGE LEVEL (MEDIUM) | 302 (EDGE PORTION 02) | CLASS 302 CORRESPONDING NOISE INTENSITY — FILTER SIZE LUT |
| | EDGE LEVEL (STRONG) | 303 (EDGE PORTION 03) | CLASS 303 CORRESPONDING NOISE INTENSITY — FILTER SIZE LUT |

## FIG. 17

PROCESSING START

S201

ESTIMATE NOISE LEVELS OF
VISIBLE LIGHT IMAGE AND FLUORESCENCE IMAGE

S202

CALCULATE NOISE REDUCTION INTENSITY $\beta$ OF
NOISE REDUCTION PROCESSING EXECUTED ON
FLUORESCENCE IMAGE SO AS TO ADJUST
NOISE LEVEL OF FLUORESCENCE IMAGE TO BE
THE SAME AS NOISE LEVEL OF VISIBLE LIGHT IMAGE

S203b

APPLY FILTER SELECTED ON THE BASIS OF CALCULATED
NOISE REDUCTION INTENSITY $\beta$ AND CLASS SET BY
PRIOR LEARNING PROCESSING, EXECUTE NOISE REDUCTION
PROCESSING ON FLUORESCENCE IMAGE, AND
GENERATE NOISE REDUCED FLUORESCENCE IMAGE

S204

SUPERIMPOSE VISIBLE LIGHT IMAGE AND NOISE REDUCED
FLUORESCENCE IMAGE ACCORDING TO SUPERIMPOSING
RATE $\alpha$ AND GENERATE SUPERIMPOSED IMAGE

PROCESSING END

*FIG. 18*

## FIG. 19

FLUORESCENCE IMAGE NOISE REDUCTION INTENSITY CALCULATION UNIT — 304

SUPERIMPOSED IMAGE IS GENERATED AS ASSUMING THAT SUPERIMPOSING RATE BE $\alpha$ AND
SUPERIMPOSED IMAGE $= (1-\alpha)$ (VISIBLE LIGHT IMAGE) $+ (\alpha)$ (FLUORESCENCE IMAGE)

**S31** EXPRESS PIXEL VALUE $y_V$ OF VISIBLE LIGHT IMAGE BY USING ORIGINAL SIGNAL $x_V$ AND NOISE COMPONENT $n_V$ AS FOLLOWS.
$$y_V = x_V + n_V$$

**S32** EXPRESS PIXEL VALUE $y_F$ OF FLUORESCENCE IMAGE BY USING ORIGINAL SIGNAL $x_F$ AND NOISE COMPONENT $n_F$ AS FOLLOWS.
$$y_F = x_F + n_F$$

**S33** EXPRESS PIXEL VALUE $y_{Fnr}$ OF FLUORESCENCE IMAGE AFTER NOISE REDUCTION BY USING NOISE REDUCTION INTENSITY $\beta$ AS FOLLOWS (HOWEVER, $\beta \geq 1$).
$$y_{Fnr} = x_F + \frac{1}{\beta} n_F$$

**S34** EXPRESS SUPERIMPOSED IMAGE BY USING THE SUPERIMPOSING RATE $\alpha$ AS FOLLOWS (HOWEVER, $0 \leq \alpha < 1$).
$$y_{nr} = (1 - \alpha) y_V + \alpha y_{Fnr}$$
$$= (1 - \alpha) x_V + \alpha x_F + (1 - \alpha) n_V + \frac{\alpha}{\beta} n_F$$

**S35** FOLLOWING FORMULA MAY BE USED FOR MAKING NOISE COMPONENT OF VISIBLE LIGHT AND NOISE COMPONENT OF FLUORESCENCE IN SUPERIMPOSED IMAGE BE EQUAL TO EACH OTHER.
$$(1 - \alpha) n_V = \frac{\alpha}{\beta} n_F$$

**S36** IN A CASE WHERE NOISE LEVEL OF VISIBLE LIGHT IMAGE IS LARGER THAN THRESHOLD (th), OUTPUT PREDETERMINED NOISE REDUCTION INTENSITY $\gamma$ TO VISIBLE LIGHT IMAGE NOISE REDUCTION UNIT AND DETERMINE NOISE REDUCTION INTENSITY $\beta$ RELATIVE TO FLUORESCENCE IMAGE FROM FORMULA INDICATED IN STEP S35 BY USING RATIO OF NOISE LEVELS OF VISIBLE LIGHT IMAGE AND FLUORESCENCE IMAGE ACCORDING TO FOLLOWING FORMULA.
$$\beta = \frac{\alpha}{1 - \alpha} \frac{n_F}{\frac{1}{\gamma} n_V}$$

FIG. 20

PROCESSING START

ESTIMATE NOISE LEVELS OF
VISIBLE LIGHT IMAGE AND FLUORESCENCE IMAGE — S301

NOISE AMOUNT > THRESHOLD
OF VISIBLE LIGHT IMAGE? — S302

**No →** S321

CALCULATE NOISE REDUCTION INTENSITY $\beta$ OF NOISE REDUCTION
PROCESSING EXECUTED ON FLUORESCENCE IMAGE SO AS TO ADJUST
NOISE LEVEL OF FLUORESCENCE IMAGE TO BE THE SAME AS NOISE LEVEL
OF VISIBLE LIGHT IMAGE WITH NO NOISE REDUCTION PROCESSING

S322

APPLY CALCULATED NOISE REDUCTION INTENSITY $\beta$, EXECUTE
NOISE REDUCTION PROCESSING ON FLUORESCENCE IMAGE,
AND GENERATE NOISE REDUCED FLUORESCENCE IMAGE

S323

SUPERIMPOSE VISIBLE LIGHT IMAGE WITH NO NOISE REDUCTION
AND NOISE REDUCED FLUORESCENCE IMAGE ACCORDING TO
SUPERIMPOSING RATE $\alpha$ AND GENERATE SUPERIMPOSED IMAGE

**Yes** S311

REDUCE NOISE OF VISIBLE LIGHT IMAGE
(APPLY PREDETERMINED NOISE REDUCTION INTENSITY $\gamma$)

S312

CALCULATE NOISE REDUCTION INTENSITY $\beta$ OF NOISE REDUCTION
PROCESSING EXECUTED ON FLUORESCENCE IMAGE SO
AS TO ADJUST NOSE LEVEL OF FLUORESCENCE IMAGE TO BE
THE SAME AS NOISE LEVEL OF VISIBLE LIGHT IMAGE ON WHICH
NOISE REDUCTION PROCESSING HAS BEEN EXECUTED

S313

APPLY CALCULATED NOISE REDUCTION INTENSITY $\beta$, EXECUTE
NOISE REDUCTION PROCESSING ON FLUORESCENCE IMAGE, AND
GENERATE NOISE REDUCED FLUORESCENCE IMAGE

S314

SUPERIMPOSE VISIBLE LIGHT IMAGE FROM WHICH NOISE HAS BEEN
REDUCED AND NOISE REDUCED FLUORESCENCE IMAGE ACCORDING TO
SUPERIMPOSING RATE $\alpha$ AND GENERATE SUPERIMPOSED IMAGE

PROCESSING END

EP 3 590 404 A1

# FIG. 21

EP 3 590 404 A1

## INTERNATIONAL SEARCH REPORT

International application No.

PCT/JP2018/004982

### A. CLASSIFICATION OF SUBJECT MATTER
Int. Cl. A61B1/00(2006.01)i, A61B1/045(2006.01)i

According to International Patent Classification (IPC) or to both national classification and IPC

### B. FIELDS SEARCHED

Minimum documentation searched (classification system followed by classification symbols)
Int. Cl. A61B1/00, A61B1/045

Documentation searched other than minimum documentation to the extent that such documents are included in the fields searched
Published examined utility model applications of Japan    1922-1996
Published unexamined utility model applications of Japan  1971-2018
Registered utility model specifications of Japan          1996-2018
Published registered utility model applications of Japan  1994-2018

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2007-75198 A (PENTAX CORP.) 29 March 2007, paragraphs [0032]-[0067], fig. 1, 6-9 & US 2007/0073104 A1, paragraphs [0044]-[0088], fig. 1, 6-9 | 1-14 |
| A | JP 2011-199786 A (OLYMPUS CORP.) 06 October 2011, paragraphs [0045]-[0067], fig. 6-13 (Family: none) | 1-14 |
| A | WO 2015/025640 A1 (OLYMPUS CORP.) 26 February 2015, paragraphs [0023]-[0076], fig. 1-13 & US 2016/0157722 A1, paragraphs [0028]-[0092], fig. 1-13 & CN 105473049 A | 1-14 |

☒ Further documents are listed in the continuation of Box C.　　　☐　See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 27.04.2018 | 15.05.2018 |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**EP 3 590 404 A1**

**INTERNATIONAL SEARCH REPORT**

| International application No. |
| --- |
| PCT/JP2018/004982 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2015-19863 A (GE MEDICAL SYSTEMS GLOBAL TECHNOLOGY CO., L.L.C.) 02 February 2015, paragraphs [0021]-[0051], fig. 1-4 (Family: none) | 1-14 |
| A | JP 2015-29841 A (MITSUBISHI ELECTRIC ENG) 16 February 2015, entire text, all drawings & US 2015/0042774 A1, entire text, all drawings | 1-14 |

Form PCT/ISA/210 (continuation of second sheet) (January 2015)

53

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013212323 A